# EUROPEAN PATENT SPECIFICATION

(11) **EP 0 943 003 B1**
(45) Date of publication and mention of the grant of the patent: **17.11.2004**
(21) Application number: 97950737.3
(22) Date of filing: 01.12.1997
(51) Int. Cl.: C12N 15/85, C07K 14/65, A61K 48/00, A01K 67/027

(54) **INSULIN-LIKE GROWTH FACTOR I (IGF-I) EXPRESSION SYSTEM AND METHODS OF USE**
INSULINÄHNLICHER WACHSTUMFAKTOR I (IGF-I) EXPRESSIONSSYSTEM UND METHODE ZUR VERWENDUNG
SYSTEME D'EXPRESSION DU FACTEUR DE CROISSANCE INSULINOIDE I (IGF-I) ET SES METHODES D'UTILISATION

(30) Priority: 02.12.1996 US 31539 P; 19.11.1997 US 974572
(43) Date of publication of application: 22.09.1999
(73) Proprietor: Valentis Inc., The Woodlands, TX 77381-4248 (US); THE BAYLOR COLLEGE OF MEDICINE, Houston, TX 77030-3498 (US)
(72) Inventor: COLEMAN, Michael, The Woodlands, TX 77381 (US); SCHWARTZ, Robert, Houston, TX 77005 (US); DEMAYO, Francesco, J., Houston, TX 77025 (US)
(74) Representative: Harrison Goddard Foote
(86) International application number: PCT/US1997/021852
(87) International publication number: WO 1998/024922

(56) References cited:
- WO-A-93/09236
- COLEMAN M E ET AL: "Myogenic Vector Expression of Insulin -like Growth Factor I Stimulates Muscle Cell Differentiation and Myofiber Hypertrophy in Transgenic Mice." JOURNAL OF BIOLOGICAL CHEMISTRY 270 (20). 1995. 12109-12116. ISSN: 0021-9258, XP000611917
- DRAGHIA-AKLI R ET AL: "Enhanced growth by ectopic expression of growth hormone releasing hormone using an injectable myogenic vector." NATURE BIOTECHNOLOGY 15 (12). 1997. 1285-1289. ISSN: 1087-0156, XP002060954
- WANG J ET AL: "Targeted overexpression of IGF-I evokes distinct patterns of organ remodeling in smooth muscle cell tissue beds of transgenic mice." JOURNAL OF CLINICAL INVESTIGATION 100 (6). 1997. 1425-1439. ISSN: 0021-9738, XP002060955

## Description

### Background of the Invention

This invention relates to vectors which encode stable messenger RNA (mRNA) and methods of using such vectors. In particular, this invention relates to vectors which establish controlled expression of recombinant genes within a tissue, which may be at levels which are useful for gene therapy and other applications. The invention further relates to vectors able to express insulin-like growth factor I (IGF-I).

IGF-I and IGF-II are low molecular weight polypeptide hormones that stimulate growth and differentiation of many cell types, including myoblasts, nerve cells, fibroblasts, chondrocytes, osteoblasts, endothelial cells, and keratinocytes (Daughaday & Rotwein, 1989, *Endocrine Reviews* 10:68-91). Expression of IGF-I is normally induced by growth hormone, and many of the growth-promoting activities of growth hormone are mediated by IGF-I (formerly called somatomedin). IGFs circulate bound to high molecular weight binding proteins that function to increase circulating half-life, inhibit insulin mimetic effects, and modulate biological activities mediated via binding to the type I IGF receptor. IGF-I has a primary role in promoting the differentiation and growth of skeletal muscle. IGFs are key myogenic progression factors which propel myoblast cell division and fusion as well as stimulate late stage muscle growth and hypertrophy. Studies by Florini (Florini & Magri, 1989, Am. J. Physiol. (Cell Physiol.) 256:C701-C711) indicate that myogenesis is stimulated by IGFs. During the onset of fusion, the biosynthesis and secretion of IGFI/II and IGF binding proteins is naturally increased in myoblasts (Tollefsen et al., 1989, *J. Biol. Chem.* 264:13810-13817). This coincides with the appearance of muscle-specific gene products.

It was shown (Coleman et al., 1995, *J. Biol. Chem*. 270:12109-12116) that increased biosynthesis and secretion of IGF-I, directed by a myogenic expression vector in C2C12 myoblasts cultures strongly stimulates the expression of myogenic helix-loop-helix factors, MyoD, myogenis, desmin, and skeletal actin mRNAs. Conversely, antisense oligodeoxynucleotides complementary to the 5' sequences of IGF-I or IGF-II mRNA were observed to suppress spontaneous differentiation in myogenic cell lines and the induction of myogenin (Florini et al, 1991, *Molecular Endocrinology* 5:718-724).

Direct evidence that IGF-I plays a role in muscle development was found when the single copy murine IGF-I gene was inactivated by homologous recombination (Powell-Braxton et al., 1993, *Genes Dev.* 7:2609-2617; Liu et al., 1993, *Cell* 75:59-72). By knocking out the IGF-I gene, severe muscle dystrophy and highly reduced myofibrillar organization of the skeletal muscle of these IGF-I mutants resulted. The majority of the mice died at birth due to respiratory failure, which was probably due to incomplete maturation of the diaphragm and intercostal muscles. These observations suggest that IGF-I is a central trophic growth factor required for embryonic muscle development and growth.

Recent studies also demonstrate a role for IGF-I in post-natal muscle growth and hypertrophy. Inclusion of IGF-I in the maintenance media of primary cultures of avian myofibers has been shown to elicit larger fiber diameters, a near doubling in myosin content and increases in protein stability and synthesis compared to untreated cultures (Vandenburg et al., 1991, Am. J. Physiol. 260:C475-C484). Administration of growth hormone to hypophysectomized rats resulted in a significant increase in IGF-I mRNA and a 50% increase in the mass of certain muscles (Englemann et al., 1989, *Mol. Cell. Endocrin.* 63:1-14). When the expression of IGF-I genes was increased through passive mechanical stretch and acute exercise, a corresponding increase in muscle hypertrophy was seen (Elgin et al., 1987, *Proc. Nat. Acad. Sci. USA* 84:3254-3258).

Studies also suggest that IGF-I is important in muscle regeneration and repair. The characteristics of regeneration vary with the injury, but invariably involves proliferation of muscle precursor cells (MPC), fusion into myotubes, and reinnervation of the muscle. During muscle regeneration, IGF-I acts as a powerful stimulant of MPC proliferation and differentiation (Grounds, 1991, Path. *Res. Pract.* 187:1-22). Studies indicate that IGF-I is produced in satellite cells and nerves within 24 hours following muscle injury and remains elevated for several weeks. In regenerating rodent muscle, the pattern of IGF-I mRNA in damaged muscle parallels muscle precursor replication from the onset (18-24 hr) to the peak (5 days).

Reactive nerve sprouting is a wide-spread phenomenon in the nervous system. Nerve sprouting is believed to be initiated by locally activating factors. Intramuscular nerve sprouting can be detected about 4 days after muscle inactivation by crush denervation. Recent studies of Caroni and Schneider, 1994, *J*. *Neurosci.* 14:3378-3388, indicate that IGF-I is required for the induction of nerve sprouting. Studies also suggest that overexpression of IG-I *in vivo* may by sufficient to enhance nerve sprouting.

### Summary of the Invention

The present invention is based in part on the identification of certain nucleic acid sequences which confer advantageous tissue targeting, expression, and secretion properties. Such sequences are utilized in the construction of plasmid vectors encoding IGF-I, for delivery and expression of the IGF-I coding sequences.

Expression of these vectors can be tissue specific. These vectors are useful in facilitating enhanced expression in tissues as well as in targeting expression with tissue specificity. These vectors can be used to treat diseases by gene therapy by restricting expression of a gene encoded on the vector to targeted tissues. Vectors containing such sequences are able to provide gene delivery and controlled expression of recombinant genes within tissues; such expression can be at certain levels that are useful for gene therapy and other applications. These vectors can also be used to create transgenic animals for research or livestock improvement.

The ability of the expression vector to provide enhanced product secretion as well as direct expression to specific tissues allows the vector to be used for treating numerous diseases. The above vectors can be used in gene therapy where a vector encoding a therapeutic product is introduced into a tissue so that tissue will express the therapeutic product. For example, the above vectors may be used for treating muscle atrophy associated with neurological, muscular, or systemic disease or aging by causing tissues to express certain trophic factors. The above vectors may be used for treating hemophilias by causing tissues to express certain clotting factors and secrete these factors into the circulation. Furthermore, the vectors above can be used for preventing or treating atherogenesis and atherosclerotic cardiovascular, cerebrovascular, or peripheral-vascular disease by causing tissue to express certain factors involved in lipid metabolism.

In addition, the vectors above can be used for gene replacement of inherited genetic defects or acquired hormone deficiencies such as diabetes, for vaccination in humans or animals to induce immune responses, or for creating transgenic animals. The transgenic animals can be used as models for studying human diseases, for assessing and exploring novel therapeutic methods, to assess the effects of chemical and physical carcinogens, and to study the effect of various genes and genetic regulatory elements. Furthermore, transgenic animals can be used to develop commercially important livestock species. The above vectors can also be used to transform cells to produce particular proteins and RNA in vitro.

Expression of such vectors having an IGF-I encoding sequence in the body of a vertebrate, *e.g.*, a human, can produce both direct and indirect effects. The IGF-I produces direct effects by the direct action of the IGF-I polypeptide. However, indirect effects may also be produced due to the effect of the IGF-I inducing or turning on the expression of other genes.

In a first aspect, the present invention features a vector for expression of a nucleic acid sequence in tissue by encoding stable mRNA. The vector includes a 5' flanking region which includes necessary sequences for the expression of a nucleic acid cassette, which include a promoter sequence from an actin gene promoter sequence. The vector also includes a 3' flanking region, which includes a 3'UTR and/or a 3' NCR from the 3' region of a growth hormone gene, which enhances secretion of the product expressed from the nucleic acid cassette. Preferably the 3' UTR is from a human growth hormone gene. Alternatively, in related vectors, the 3' sequences may be selected to provide a higher level of retention of the product within a tissue, *e.g.*, within a muscle tissue, rather than enhancing secretion. Such sequences can, for example, be from a skeletal α-actin gene. The vector also includes a linker which connects the 5' flanking region to a nucleic acid. The linker does not contain the coding sequence of a gene that the linker is naturally associated with. That is, the linker is not the normal gene associated with the 5' and 3' regions. Preferably, the linker includes a sequence coding for an IGF-I gene, more preferably human IGF-I. The 3' flanking region is 3' to the position for inserting the nucleic acid cassette.

The term "flanking region" as used herein refers to nucleotide sequences on either side of an associated gene. Flanking regions can be either 3' or 5' to a particular gene in question. In general, flanking sequences contain elements necessary for regulation of expression of a particular gene. Such elements include, but are not limited to, sequences necessary for efficient expression, as well as tissue specific expression. Examples of sequences necessary for efficient expression can include specific regulatory sequences or elements adjacent to or within the protein coding regions of DNA. These elements, located adjacent to the gene, are termed cis-acting elements. The signals are recognized by other diffusible biomolecules in *trans* to alter the transcriptional activity. These biomolecules are termed trans-acting factors. *Trans*-acting factors and *cis*-acting elements have been shown to contribute to the timing and developmental expression pattern of a gene. *Cis*-acting elements are usually thought of as those that regulate transcription and are usually found within promoter regions and within upstream (5') or downstream (3') DNA flanking regions.

Flanking DNA with regulatory elements that regulate expression of genes in tissue may also include modulatory or regulatory sequences which are regulated by specific factors, such as glucocorticoids, androgens, progestins, antiprogestins (PCT US93/04399; PCT US96/04324), vitamin D₃ and its metabolites, vitamin A and its metabolites, retinoic acid, calcium as well as others.

"Modulatory" or "regulatory" sequences as used herein refer to sequences which may be in the 3' or 5' flanking region, where such sequences can enhance activation and/or suppression of the transcription of the associated gene.

"Responsive" or "respond" as used herein refers to the enhancement of activation and/or suppression of gene transcription as discussed below.

"Metabolite" as used herein refers to any product from the metabolism of the regulatory factors which regulate gene expression.

In addition to the above, either or both of the 3' or 5' flanking regions can cause tissue specificity. Such tissue specificity provides expression predominantly in a specified cell or tissue.

"Predominantly" as used herein means that the gene associated with the 3' or 5' flanking region is expressed to a higher degree only in the specific tissue as compared to low expression or lack of expression in nonspecific tissue. The 3' or 5' flanking regions singularly or together as used herein can provide expression of the associated gene in other tissues but to a lower degree than expression in tissues or cells where expression is predominate. Expression is preferentially in the specified tissue. Such predominant expression can be compared with the same magnitude of difference as will be found in the natural expression of the gene (i.e. as found in a cell in vivo) in that particular tissue or cell type as compared with other nonspecific tissues or cells. Such differences can be observed by analysis of mRNA levels or expression of natural gene products, recombinant gene products, or reporter genes. Furthermore, northern analysis, X gal immunofluorescence or CAT assays as discussed herein and as known in the art can be used to detect such differences.

The 3' flanking region contains sequences or regions, e.g. 3'UTR and/or 3' NCR, which regulate expression of a nucleic acid sequence with which it is associated. The 3' flanking regions can provide tissue-specific expression to an associated gene. The 3' flanking region also contains a transcriptional termination signal.

The term "3' flanking region" as used herein includes that portion of a naturally occurring sequence 3' to the transcribed portion of the gene which are responsible for mRNA processing and/or tissue-specific expression. That portion can be readily defined by known procedures. For example, the portions of a 3' flanking region which are responsible for mRNA stability and/or tissue-specific expression can be mapped by mutational analysis or various clones created to define the desired 3' flanking region activity in a selected vector system.

The 3' flanking region can contain a 3'UTR and/or a 3' NCR. The term "3' untranslated region" or "3'UTR" refers to the sequence at the 3' end of structural gene which is transcribed from the DNA but not translated into protein. This 3'UTR region does not contain a poly(A) sequence, but generally contains a site at which a poly(A) sequence is added. Poly (A) sequences are only added after the transcriptional process.

Myogenic-specific 3'UTR sequences can be used to allow for specific stability in muscle cells or other tissues. As described below, myogenic-specific sequences refers to gene sequences normally expressed in muscle cells, e.g., skeletal, heart and smooth muscle cells. Myogenic specific mRNA stability provides an increase in mRNA stability within myogenic cells. The increase in stability provides increased expression. The 3'UTR and 3' NCR sequences singularly or together can provide a higher level of mRNA accumulation through increased mRNA stability. Thus, increased expression and/or stability of mRNA leads to increased levels of protein production.

The term "3' non-coding region" or "3'NCR" is a region which is adjacent to the 3'UTR region of a structural gene. The 3'NCR region generally contains a transcription termination signal. Once transcription occurs and prior to translation, the RNA sequence encoded by the 3'NCR is usually removed so that the poly(A) sequence can be added to the mRNA. The 3'NCR sequences can also be used to allow mRNA stability as described above. The 3'NCR may also increase the transcription rate of the nucleic acid cassette.

Either or both of the 3'UTR and 3' NCR sequences can be selected from a group of myogenic-specific genes. Examples of myogenic-specific genes include the skeletal α-actin gene, fast myosin-light chain 1/3 gene, myosin-heavy chain gene, troponin T gene, acetylcholine receptor subunit genes and muscle creatinine kinase gene.

In reference to 3' flanking regions of this invention, the term "growth hormone" refers to a gene product identified as a growth hormone, for example, human growth hormone or bovine growth hormone. Homologous gene sequences are known in the art for a variety of different vertebrate animals. In different embodiments, the vectors can incorporate 3' sequences, including 3' UTR sequences from such growth hormone genes. The 3' sequence can be modified from the sequence naturally found in the animal, for example by the deletion of ALU repeat sequence from human growth hormone 3' UTR. The deletion of ALU repeats or ALU repeat-like sequences can be performed with a variety of 3' sequences; such deletion generally reduces the rate of homologous recombination. A variety of other modifications may also be made without destroying the tissue targeting, stabilizing, and secretion properties of the 3' sequence.

The 5' flanking region is located 5' to the associated gene or nucleic acid sequence to be expressed. Just as with the 3' flanking region, the 5' flanking region can be defined by known procedures. For example, the active portion of the 5' flanking region can be mapped by mutational analysis or various clones of the 5' flanking region created to define the desired activity in a selected vector. The 5' flanking region can include, in addition to the above regulatory sequences or elements, a promoter, a TATA box, and a CAP site, which are in an appropriate relationship sequentially and positionally for the expression of an associated gene.

In this invention, "sequences necessary for expression" are those elements of the 5' flanking region which are sequentially and positionally in an appropriate relationship to cause controlled expression of a gene within a nucleic acid cassette. Expression is controlled to certain levels within tissues such that the expressed gene is useful for gene therapy and other applications involving gene delivery. The 5' sequence can contain elements which regulate tissue-specific expression or can include portions of a naturally occurring 5' element responsible for tissue-specific expression.

The term "promoter," as used herein refers to a recognition site on a strand of DNA to which RNA polymerase binds. The promoter usually is a DNA fragment of about 100 to about 200 base pairs (in eukaryotic genes) in the 5' flanking DNA upstream of the CAP site or the transcriptional initiation start site. The promoter forms an "initiation complex" with RNA polymerase to initiate and drive transcriptional activity. The complex can be modified by activating sequences termed "enhancers" or inhibitory sequences termed "silencers". The promoter can be one which is naturally (i.e., associated as if it were within a cell *in vivo*) or non-naturally associated with a 5' flanking region.

A variety of promoters can be used. Some examples include thymidine kinase promoter, myogenic-specific promoters including skeletal α-actin gene promoter, fast myosin light chain 1 promoter, myosin heavy chain promoter, troponin T promoter, and muscle creatinine kinase promoter, as well as non-specific promoters including the cytomegalovirus immediate early promoter, Rous Sarcoma virus LTR. These promoters or other promoters used with the present invention can be mutated in order to increase expression of the associated gene. Furthermore a promoter may be used by itself or in combination with elements from other promoters, as well as various enhancers, transcript stabilizers, or other sequences capable of enhancing function of the vector.

"Mutation" as used herein refers to a change in the sequence of genetic material from normal causing a change in the functional characteristics of the gene. This includes gene mutations where only a single base is changed in the natural gene promoter sequences or multiple bases are changed.

The term "intron" as used herein refers to a section of DNA occurring in a transcribed portion of a gene which is included in a precursor RNA but is then excised during processing of the transcribed RNA before translation occurs. Intron sequences are therefore not found in mRNA nor translated into protein. The term "exon" as used herein refers to a portion of a gene that is included in a transcript of a gene and survives processing of the RNA in the cell to become part of a mature mRNA. Exons generally encode three distinct functional regions of the RNA transcript. The first, located at the 5' end which is not translated into protein, termed the 5' untranslated region (5' UTR), signals the beginning of RNA transcription and contains sequences that direct the mRNA to the ribosomes and cause the mRNA to be bound by ribosomes so that protein synthesis can occur. The second contains the information that can be translated into the amino acid sequence of the protein or function as a bioactive RNA molecule. The third, located at the 3' end is not translated into protein, i.e. 3' UTR, contains the signals for termination of translation and for the addition of a polyadenylation tail (poly(A). In particular, the 3' UTR as defined above can provide mRNA stability. The intron/exon boundary will be that portion in a particular gene where an intron section connects to an exon section. The terms "TATA box" and "CAP site" are used as they are recognized in the art.

The term "linker" as used herein refers to DNA which contains the recognition site for a specific restriction endonuclease. Linkers may be ligated to the ends of DNA fragments prepared by cleavage with some other enzyme. In particular, the linker provides a recognition site for inserting the nucleic acid cassette which contains a specific nucleic sequence to be expressed. This recognition site may be but is not limited to an endonuclease site in the linker, such as Cla-I, Not-I, Xmal, Bgl-II, Pac-I, Xhol, Nhel, Sfi-I. A linker can be designed so that the unique restriction endonuclease site contains a start codon (e.g. AUG) or stop codon (e.g. TAA, TGA, TCA) and these critical codons are reconstituted when a sequence encoding a protein is ligated into the linker. Such linkers commonly include an NcoI or SphI site.

The term "leader" as used herein refers to a DNA sequence at the 5' end of a structural gene which is transcribed and translated along with the gene. The leader usually results in the protein having an n-terminal peptide extension sometimes called a pro-sequence. For proteins destined for either secretion to the extracellular medium or the membrane, this signal sequence directs the protein into endoplasmic reticulum from which it is discharged to the appropriate destination. The leader sequence normally is encoded by the desired nucleic acid, synthetically derived or isolated from a different gene sequence. A variety of leader sequences from different proteins can be used in the vectors of the present invention. Some non-limiting examples include gelsolin, albumin, fibrinogen and other secreted serum proteins.

The term "vector" as used herein refers to a nucleic acid, e.g., DNA derived from a plasmid, cosmid, phasmid or bacteriophage or synthesized by chemical or enzymatic means, into which one or more fragments of nucleic acid may be inserted or cloned which encode for particular genes. The vector can contain one or more unique restriction sites for this purpose, and may be capable of autonomous replication in a defined host or organism such that the cloned sequence is reproduced. The vector may have a linear, circular, or supercoiled configuration and may be complexed with other vectors or other materials for certain purposes. The components of a vector can include but are not limited to a DNA molecule incorporating: (1) a sequence encoding a therapeutic or desired product; and (2) regulatory elements for transcription, translation, RNA stability and replication.

The vector can be used to provide expression of a nucleic acid sequence in tissue. In the present invention this expression is enhanced by providing stability to an mRNA transcript from the nucleic acid sequence and/or secretion of the therapeutic protein. Expression includes the efficient transcription of an inserted gene or nucleic acid sequence within the vector. Expression products may be proteins including but not limited to pure protein (polypeptide), glycoprotein, lipoprotein, phosphoprotein, or nucleoprotein. Expression products may also be RNA. The nucleic acid sequence is contained in a nucleic acid cassette. Expression of the nucleic acid can be continuous or controlled by endogenous or exogenous stimuli.

The term "control" or "controlled" as used herein relates to the expression of gene products (protein or RNA) at sufficiently high levels such that a therapeutic effect is obtained. Levels that are sufficient for therapeutic effect are lower than the toxic levels. Levels of expression for therapeutic effect within selected tissues corresponds to reproducible kinetics of uptake, elimination from cell, post-translational processing, and levels of gene expression, and, in certain instances, regulated expression in response to certain endogenous or exogenous stimuli (e.g., hormones, drugs).

The term "nucleic acid cassette" as used herein refers to the genetic material of interest which codes for a protein or RNA. The nucleic acid cassette is positionally and sequentially oriented within the vector such that the nucleic acid in the cassette can be transcribed into RNA, and when necessary, translated into a protein in the transformed tissue or cell. Preferably, the cassette has 3' and 5' ends adapted for ready insertion into a vector, *e.g.*, it has restriction endonuclease sites at each end. In the vectors of this invention, a nucleic acid cassette contains a sequence coding for insulin-like growth factor I (IGF-I), *e.g.*, human IGF-I.

The term "tissue" as used herein refers to a collection of cells specialized to perform a particular function or can include a single cell. The cells may be of the same type or of different types.

The term "gene", e.g., "myogenic genes," as used herein refers to those genes exemplified herein and their equivalence in other animal species or other tissues. Homologous sequences (i.e. sequences having a common evolutionary origin representing members of the same superfamily) or analogous sequences (i.e. sequences having common properties though a distinct evolutionary origin) are also included so long as they provide equivalent properties to those described herein. It is important in this invention that the chosen sequence provide the enhanced levels of expression, expression of the appropriate product, and/or tissue-specific expression as noted herein. Those in the art will recognize that the minimum sequences required for such functions are encompassed by the above definition. These minimum sequences are readily determined by standard techniques exemplified herein.

The term "myogenic" refers to muscle tissue or cells. The muscle tissue or cells can be *in vivo, in vitro,* or *in vitro* tissue culture and capable of differentiating into muscle tissue. Myogenic cells include skeletal, heart and smooth muscle cells. Genes are termed "myogenic" or "myogenic-specific" if they are expressed or expressed preferentially in myogenic cells. Vectors are termed "myogenic" or "myogenic-specific" if they function preferentially in myogenic muscle tissue or cells. Myogenic activity of vectors can be determined by transfection of these vectors into myogenic cells in culture, injected into intact muscle tissue, or injected into mammalian oocytes to be stably incorporated into the genome to generate transgenic animals which express the protein or RNA of interest in myogenic cells.

The term "non-myogenic" refers to tissue or cells other than muscle. The tissues or cells can be *in vivo, in vitro,* or in vitro tissue culture.

In a preferred embodiment, the vector described above may have its 5' flanking region from myogenic genes, in particular the skeletal α-actin gene, e.g., a chicken skeletal α-actin gene. Specifically, this can include a promoter sequence which may be linked with other 5' UTR sequences, which can include an intron. While vectors using the chicken skeletal α-actin promoter and/or other 5' flanking sequences are exemplified herein, the 5' sequences for α-actin genes are highly conserved, therefore, such 5' α-actin sequences can be utilized from other vertabrate species, including, for example, human.

The 3' UTR is from a growth hormone gene, preferably from a human growth hormone gene, and preferably includes a poly(A) signal. This sequence can be linked immediately following the natural translation termination codon for a cDNA sequence coding for the protein or RNA to be expressed. As discussed above, these regions can be further and more precisely defined by routine methodology, *e*.*g*., deletion or mutation analysis or their equivalents.

The 5' or 3' sequences may have a sequence identical to the sequence as naturally found, but may also have changed sequences which provide equivalent function to a vector in which such 5' or 3' sequences are incorporated. Such a change, for example, could be a change of ten nucleotides in any of the above regions. In particular, such changes can include the deletion of ALU repeat sequences from the 3' UTR. This is only an example and is non-limiting.

Also in a preferred embodiment, the sequence encoding IGF-I is a synthetic IGF-I coding sequence. Such a synthetic sequence has a nucleotide sequence which differs from a natural human IGF-I coding sequence. It is preferred that the sequence utilize optimal codon usage; preferably at least 50%, 70%, or 90% of the codons are optimized. Thus, in preferred embodiments the synthetic DNA sequence has at least 80, 90, 95, or 99% sequence identity to the sequence of SEQ ID NO. 1. In a particular preferred embodiment, the synthetic DNA sequence has at least 95% identity, more preferably at least 99% identity, and most preferably 100% identity to the sequence of SEQ ID NO. 4.

In addition, another embodiment of the above vector may contain a regulatory system for regulating expression of the nucleic acid cassette. The term "regulatory system" as used herein refers to cis-acting or transacting sequences incorporated into the above vectors which regulate in some characteristic the expression of the nucleic acid of interest as well as trans-acting gene products which are co-expressed in the cell with the above described vector. Regulatory systems can be used for up-regulation or down regulation of expression from the normal levels of expression or existing levels of expression at the time of regulation. The system contributes to the timing and developmental expression pattern of the nucleic acid.

One construction of a regulatory system includes a chimeric trans-acting regulatory factor incorporating elements of a serum response factor capable of regulating expression of the vector in a cell. The chimeric transacting regulatory factor is constructed by replacing the normal DNA binding domain sequence of the serum response factor with a DNA binding domain sequence of a receptor. The serum response factor has a transactivation domain which is unchanged. The transactivation domain is capable of activating transcription when an agent or ligand specific to the receptor binding site binds to the receptor. Thus, regulation can be controlled by controlling the amount of the agent.

The DNA binding domain sequence of a receptor, incorporated into the chimeric trans-activating regulatory factor, can be selected from a variety of receptor groups including but not limited to vitamin, steroid, thyroid, orphan hormone, retinoic acid, thyroxine, or GAL4 receptors. The chimeric trans-activating regulator factor is usually located within the sequence of the promoter. In one preferred embodiment the promoter used in the vector is the α-actin promoter and the receptor is the vitamin D receptor.

"Receptor" as used herein includes natural receptors (i.e., as found in a cell *in vivo*) as well as anything that binds alike and causes compartmentalization changes in a cell.

Another embodiment of the regulatory system includes the construction of a vector with two functional units. One functional unit expresses a receptor. This functional unit contains elements required for expression including a promoter, a nucleic acid sequence coding for the receptor, and a 3' UTR and/or a 3' NCR. The second functional unit expresses a therapeutic protein or RNA and contains, in addition, a response element corresponding to the receptor, a promoter, a nucleic acid cassette, and a 3' UTR and/or a 3' NCR. These functional units can be in the same or separate vectors.

The first functional unit expresses the receptor. It is favorable to use a receptor not found in high levels in the target tissue. The receptor forms an interaction, e.g., ionic, non-ionic, hydrophobic, H-bonding, with the response element on the second functional unit prior to, concurrent with, or after the binding of the agent or ligand to the receptor. This interaction allows the regulation of the nucleic acid cassette expression. The receptor can be from the same nonlimiting group as disclosed above. Furthermore, the vector can be myogenic specific by using myogenic specific 3' UTR and/or 3' NCR sequences.

In an exemplary preferred embodiment the plasmid can be pIG0552 or a plasmid comprising a nucleotide sequence which is the same as the sequence of pIG0552. This is only an example and is meant to be non-limiting. Thus, sequence changes or variations can be made to one or more of the sequence elements, such as the 5' and 3' flanking regions. The sequences utilized for this exemplary vector have the advantage of providing an IGF-I RNA splice product which produces a polypeptide having a signal sequence of equal length as a form found naturally in muscle and many other tissues.

In this context, the word "same" means that the sequences are functionally equivalent and have a high degree of sequence identity. However, the sequences may have a low level of sequence differences, such as by substitution, deletion, or addition of one or more nucleotides. Such sequences will preferably be less than 10%, more preferably less than 5%, and still more preferably less than 1% of the total sequence.

In particular embodiments, the vectors of the above aspect may alternatively comprise, consist essentially of, or consist of the stated elements or sequences.

By "comprising" it is meant including, but not limited to, whatever follows the word "comprising". Thus, use of the term "comprising" indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

A related aspect of the invention provides a formulation for delivery and expression of an IGF-I gene in a cell, preferably a human IGF-I gene. The formulation includes a vector of the above aspect together with one or more other components which can, for example, act to stablilize the vector or to enhance transfection efficiency, but can also provide other functions. In a preferred embodiment, the formulation includes the vector in a solution having between about 0.5% and 50% polyvinyl pyrrolidone (PVP), preferably about 5% PVP. Preferably, the PVP has a weight average molecular weight of about 50,000 g/mol. Further information is disclosed in PCT US95/17038. However, another example of a formulation includes the vector with a cationic lipid (e.g., as described in U.S. Patent 4,897,355, issued January 30, 1990), and can also include a co-lipid, such as a neutral co-lipid.

In reference to the formulations of this invention, the term "about" indicates that in preferred embodiments, the actual value for a particular parameter is in the range of 50%-200% of the stated value.

Another related aspect of the invention features a transgenic animal, at least some cells of which contain vectors of the first aspect of the present invention. These cells include germ or somatic cells. The transgenic animals can be used as models for studying human diseases, for assessing and exploring novel therapeutic methods, to assess the effects of chemical and physical carcinogens, and to study the effect of various genes and genetic regulatory elements. In addition, transgenic animals can be used to develop commercially important livestock species.

A fourth related aspect of the present invention features cells transformed with a vector of the present invention for expression of a IGF-I nucleic acid sequence, preferably a hIGF-I nucleic acid sequence.

As used herein, "transformation" is the change in a cell's phenotypic characteristics by the action of a gene expressing a gene product. The gene causing the phenotypic characteristic change has been transfected into the cell.

The term "transfection" as used herein refers to a mechanism of gene transfer which involves the uptake of DNA by a cell or organism. Following entry into the cell, the transforming DNA may recombine with that of the host by physically integrating into the chromosomes of the host cell, may be maintained transiently as an episomal element without being replicated, or may replicate independently as a plasmid. Preferably the transforming DNA does not integrate.

Transfection can be performed by *in vivo* techniques as described below, or by *ex vivo* techniques in which cells are co-transfected with a vector containing a selectable marker. This selectable marker is used to select those cells which have become transformed. It is well known to those skilled in the art the type of selectable markers to be used with transfection/transformation studies. An example of such a marker is a neo gene, providing neomycin/kanamycin resistance. Transfection/transformation can be tissue-specific, i.e., involve the use of myogenic specific vectors which cause expression of the nucleic acid cassette predominantly in the tissue of choice. In particular, tissue specificity can be directed to myogenic cells by using a promoter and/or 3'UTR and/or 3' NCR sequences specific for myogenic tissue expression.

A fifth related aspect of the present invention feature methods for transfecting a cell *ex-vivo* with the vectors of the present invention. These methods comprise the steps of contacting a cell *in situ* with a vector of the present invention for sufficient time to transfect the cell.

A sixth related aspect of the invention provides method for *ex-vivo* delivery and expression of an IGF-I gene preferably a hIGF-I gene. The method comprises transfecting plurality of cells *ex-vivo* with a vector of the first aspect and incubating the cells under conditions allowing expressic of a nucleic acid sequence of the vector, which codes for IGF I. The "conditions allowing expression" may be any of variety of conditions. Under such conditions, the cells will produce the gene product from the vector DNA in detectabl quantities.

A seventh related aspect of the present invention features the use of the above-referenced vector as a medicament. The disease or condition may, for example, be localized or systemic. Disease can include but is not limited to muscle atrophy, atherogenesis, atherosclerotic cardiovascular, cerebrovascular, or peripheral vascular disease, diabetes, neuropathy, growth disorders and hemophilia. These vectors contain nucleic acid sequences coding for insulin-like growth factor I.

The muscle atrophy to be treated may be due to any of a variety of different causes. For example, muscle weakness may be primarily due to disuse atrophy which commonly occurs in situations such as joint replacement. Likewise, the muscle weakness and atrophy may be secondary to lower motor neuron injury. Such lower motor neuron injury can have a large number of different causes, including, for example, cubital tunnel syndrome, Bell's palsy, carpal tunnel syndrome, spinal fracture, or diabetic neuropathy. The causes may also include genetic causes of muscular atrophy, including, for example, muscular dystrophy. These causes and conditions are only exemplary and are not limiting to the invention.

Thus, "localized" disease or condition refers to those in which there is specific nerve or muscle damage or atrophy to a defined and limited area of the body. A specific example is disuse atrophy. A " systemic" disease of condition refers to those which relate to the entire organism, or is widely distributed at a number of locations within the body. Examples include diabetes, growth disorders, neuopathies, and muscular dystrophy.

The methods of treating disease of the present invention feature methods for establishing expression of IGF-I in tissue by administration of a vector. These methods of use of the above-referenced vectors comprises the steps of administering an effective amount of the vectors to a human, animal or tissue culture.

The term "administering" or "administration" as used herein refers to the route of introduction of a vector or carrier of DNA into the body. The vectors of the above methods and the methods discussed below may be administered by various routes. In particular a preferred target cell for treatment is the skeletal muscle cell.

The term "skeletal muscle" as used herein refers to those cells which comprise the bulk of the body's musculature, i.e., striated muscle.

Administration can be directly to a target tissue or may involve targeted delivery after systemic administration. The preferred embodiments are by direct injection into muscle or targeted uptake into muscle after intravenous injection.

The term "delivery" refers to the process by which the vector comes into contact with the preferred target cell after administration. Administration may involve needle injection into cells, tissues, fluid spaces, or blood vessels, electroporation, transfection, hypospray, iontophoresis, particle bombardment, or transplantation of cells genetically modified *ex vivo.* Examples of administration include intravenous, intramuscular, aerosol, oral, topical, systemic, ocular, intraperitoneal and/or intrathecal.

The preferred means for administration of vectors described above involves the use of formulations for delivery to the target cell in which the vector is associated with elements such as lipids, proteins, carbohydrates, synthetic organic compounds, or in-organic compounds which enhance the entry of the vector into the nucleus of the target cell where gene expression may occur. A particular example is PVP.

The term "formulation" as used herein refers to non-genetic material combined with the vector in a solution, suspension, or colloid which enhances the delivery of the vector to a tissue, uptake by cells within the tissue, intracellular trafficking through the membrane, endosome or cytoplasm into the nucleus, the stability of the vector in extracellular or intracellular compartments, and/or expression of genetic material by the cell.

In a preferred embodiment of the present invention the vector and formulation comprises a nanoparticle which is administered as a suspension or colloid. The formulation can include lipids, proteins, carbohydrates, synthetic organic compounds, or inorganic compounds. Examples of elements which are included in a formulation are lipids capable of forming liposomes, cationic lipids, hydrophilic polymers, polycations (e.g. protamine, polybrine, spermidine, polylysine), peptide or synthetic ligand recognizing receptors on the surface of the target cells, peptide or synthetic ligand capable of inducing endosomal-lysis, peptide or synthetic ligand capable of targeting materials to the nucleus, gels, slow release matrices, salts, carbohydrates, nutrients, or soluble or insoluble particles as well as analogues or derivatives of such elements. This includes formulation elements enhancing the delivery, uptake, stability, and/or expression of genetic material into cells. This list is included for illustration only and is not intended to be limiting in any way.

Another embodiment of the present invention features the above vectors with coating elements that enhance expression as well as uptake by the cell. The term "coating" as used herein refers to elements, proteins or molecules used to associate with the vector in order to enhance cellular uptake. In particular, coating includes a DNA initiation complex and histones. The coating improves the stability of the vector, its entry into the nucleus, and the efficiency of transcription.

The term "DNA initiation complex" as used herein refers to a complex containing a serum response factor, a transcription initiation factor and a transregulatory factor. The serum response factor is attached to or interacts with the serum response element within the promoter region of the vector. The transcription initiation factor and the transregulatory factor then interact with the serum response factor and the promoter, in particular the TATA box within the promoter, to form a stable DNA complex. The term "histone" as used herein refers to nuclear proteins which associate with and/or bind to DNA, e.g., a vector. The histones can bind specifically or non-specifically to the DNA.

The term "effective amount" as used herein refers to sufficient vector administered to humans, animals or into tissue culture to produce the adequate levels of protein or RNA. One skilled in the art recognizes that the adequate level of protein or RNA will depend on the use of the particular vector. These levels will be different depending on the type of administration and treatment or vaccination.

The methods for treating diseases as disclosed herein includes treatment with biological products (specifically proteins as defined above) in which the disease being treated requires the protein to circulate through the body from the. general circulation. For example, disorders which might be treated by the present invention include osteoporosis by expression of IGF-I or its binding proteins. The selection of the appropriate protein to treat various diseases will be apparent to one skilled in the art.

In creating disease; the present invention provides a means for achieving: (1) sufficiently high levels of a particular protein to obtain a therapeutic effect; (2) controlled expression of product at levels which are sufficient for therapeutic effect and lower than the toxic levels; (3) controlled expression in certain tissues in order to obtain reproducible pharmacokinetics and levels of gene expression; and (4) delivery using clinically and pharmaceutically acceptable means of administration and formulation rather than transplantation of genetically engineered and selected cells.

In doing so, the present invention provides significant advances over the art. First, promoters from viral genomes and viral vectors which were used to obtain high level expression in tissue, were not able to provide controlled expression. Second, promoters from various tissue-specific genes which were used to obtain controlled expression in transgenic animals and animal models of gene therapy did not have a sufficiently high level of expression to obtain therapeutic effect. In addition, in treating diseases with the present invention, the ability to raise antibodies against protein products does not reflect the ability to achieve controlled expression of proteins within the therapeutic range.

The genetic material which is incorporated into the cells from the above vectors can be any natural or synthetic nucleic acid. For example, the nucleic acid can be: (1) not normally found in the tissue of the cells; (2) normally found in a specific tissue but not expressed at physiological significant levels; (3) normally found in specific tissue and normally expressed at physiological desired levels; (4) any other nucleic acid which can be modified for expression in skeletal muscle cells; and (5) any combination of the above. In addition to the genetic material which is incorporated into tissue, the above reference is also applicable to genetic material which is incorporated into a cell.

Other features and advantages of the invention will be apparent from the following detailed description of the invention in conjunction with the accompanying drawings and from the claims.

### Brief Description of the Drawings

Figure 1 is a schematic drawing of the chicken skeletal α-actin gene which includes the location of certain unique restriction sites.
Figure 2 is a schematic representation of a myogenic vector system.
Figure 3 is a schematic diagram of a set of skeletal α-actin/insulin like growth factor-I/skeletal α-actin hybrid genes.
Figure 4 is a schematic representation of the exemplary plasmid pIG0552.

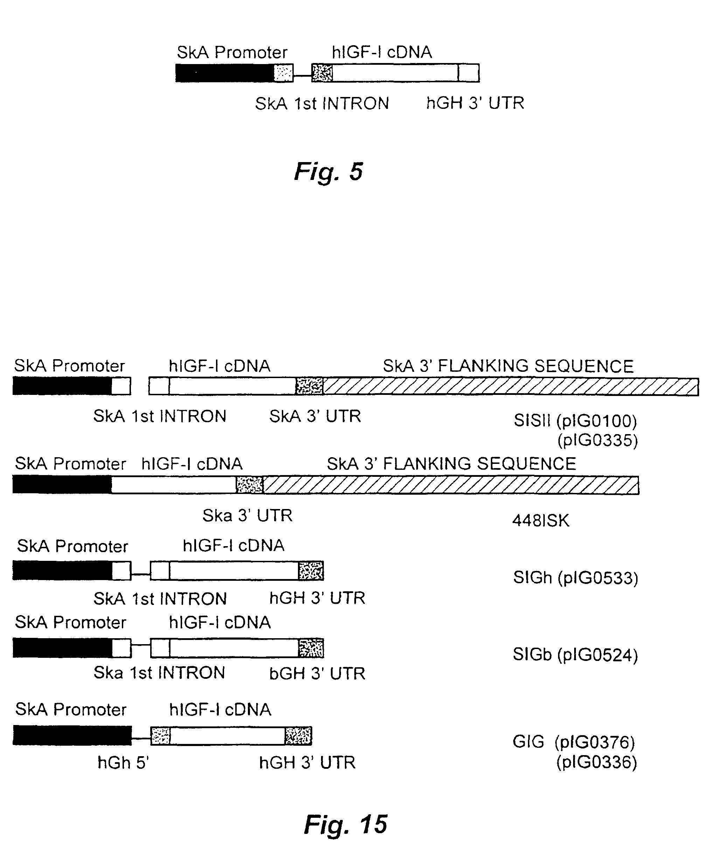

Figure 5 is a schematic drawing of an exemplary expression unit as incorporated in plasmid pIG0552. The functional elements are denoted as: SkA Promoter - 5' promoter region from chicken skeletal α-actin gene (Bergsma et al., 1986, *Mol. Cell. Biol.* 6:2462-2475); SkA 1st Intron - 5' UTR (exon 1), first intron, exon 2 up to initiation ATG from chicken skeletal α-actin gene (Coleman et al., 1995, *J*. *Biol*. *Chem.* 270:12109-12116); hIGF-I cDNA - human IGF-I cDNA (Jansen et al., 1983, *Nature* 306(8):609-611; Powell-Braxton et al., 1993, *Genes Dev.* 7(12B) :2609-2617) ; hGH 3' UTR - 3' untranslated region and poly A addition signal from human growth hormone gene.
Figures 6-8 provide a schematic representation of the construction scheme for pIG0552 showing the generalized structures of the starting and intermediate plasmids utilized in that construction process.
Figure 9 is a chart showing the codon usage frequencies for highly expressed human genes.
Figures 10A and 10B are graphs showing results from a mouse model of nerve crush. Data shown are mean +/- SEM for n=4 to 5 (sham) and n=7 to 8 (crush/control and crush/hIGF-I plasmid) animals/group/time point. Fig. 10A shows the effect of hIGF-I plasmid IM administration on nerve conduction velocity. Fig. 10B shows the effect of hIGF-I plasmid IM administration on electromyograph activity (EMG).

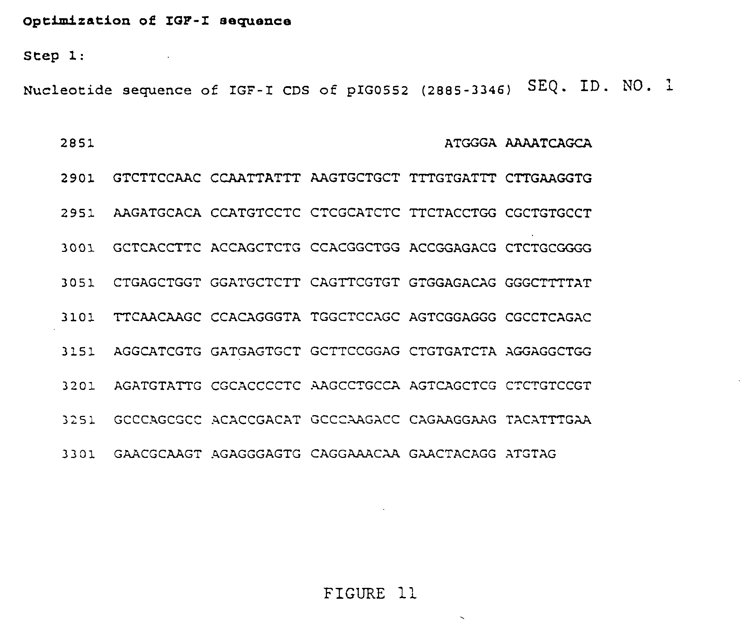

Figure 11 provides the sequence encoding hIGF-I as incorporated in pIG0552 (SEQ ID NO. 1).

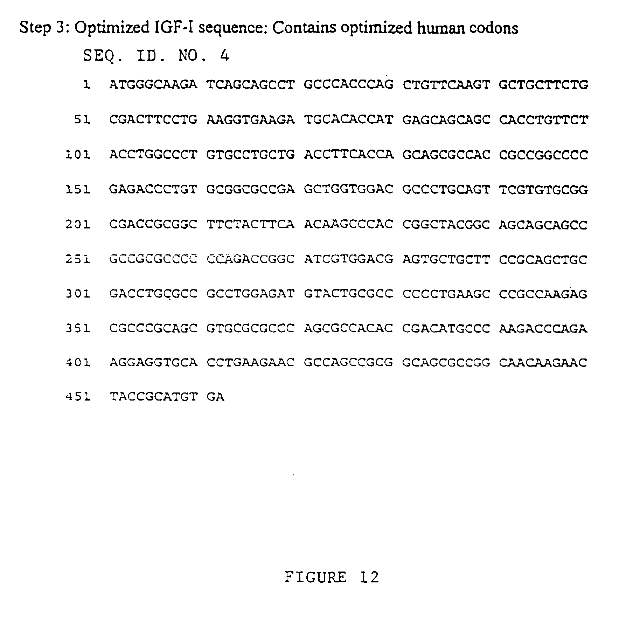

Figure 12 provides the sequence encoding hIGF-I having optimized codon usage (SEQ ID NO. 4).

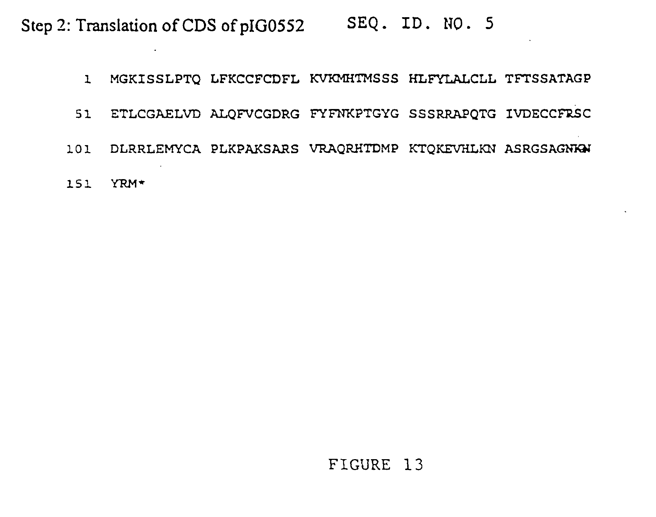

Figure 13 provides the amino acid sequence encoded by both SEQ ID NOS. 1 & 4.

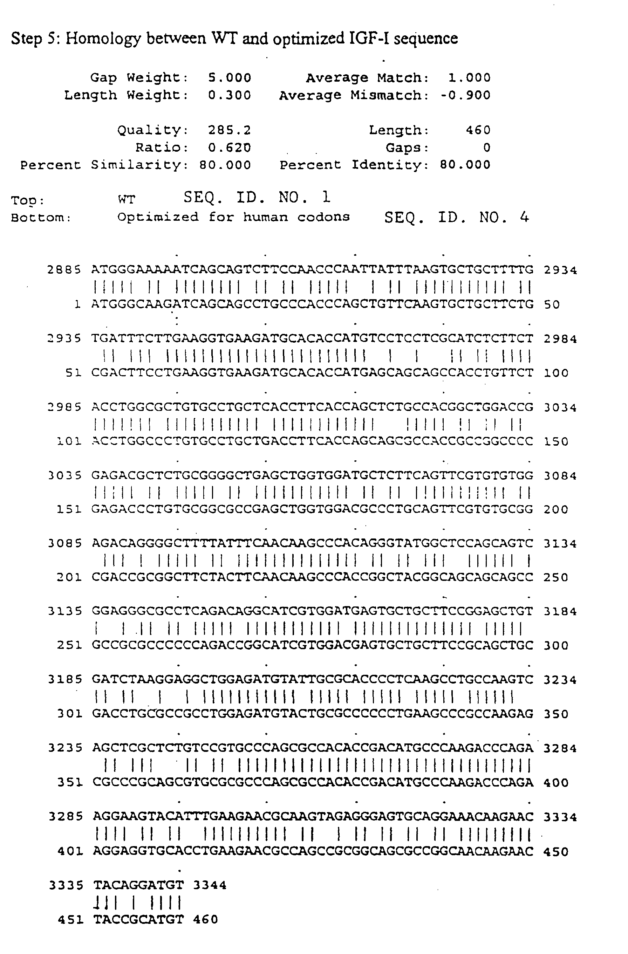

Figure 14 is an alignment of the hIGF-I coding sequences (SEQ ID NOS. 1 & 4), showing the positions of the nucleotide differences between the two sequences. As indicated, the sequences have approximately 80% identity.
Figure 15 schematically shows transgenes encoding hIGF-I used in transgenic mice.

The drawings are not necessarily to scale, and certain features of the invention may be exaggerated in scale and shown in schematic form in the interest of clarity and conciseness.

### Detailed Description of the Invention

The vectors and methods of this invention provide for the delivery and expression of IGF-I in mammalian cells, e.g., in human cells. It has been shown that IGF-I plays an important role in normal muscle development, muscle growth and hypertrophy, muscle regeneration and maintenance/regeneration of peripheral nerves.

The following are specific examples of preferred embodiments of the present invention and are not intended to limit the invention. These examples demonstrate how the expression vector systems of the present invention can be used in construction of various cellular or animal models, and how genes can be regulated by sequences within such vectors. The description and utility of such vectors and related vectors is discussed herein and is amplified upon in Schwartz et al., U.S. Patent No. 5,298,422, entitled "Myogenic Vector Systems,", and co-pending application Schwartz et al., Application No. 08/472,809, entitled "Expression Vector Systems and Method of Use".

Below are provided examples of specific regions of 5' UTR and 3' UTR and/or 3' NCR regions of myogenic genes that can be used to provide certain functionalities to an expression vector, and thus within a transformed cell or animal containing such a vector. Those in the art will recognize that specific portions of these regions can be identified as that containing the functional nucleic acid sequence providing the desirable property, and such regions can be readily defined using routine deletion or mutagenic techniques or their equivalent. Such regions include the promoter, enhancer and cis- and transacting elements of a regulatable system. As noted herein, such controlling segments of nucleic acid may be inserted at any location on the vector, although there may be preferable sites as described herein.

### Isolation of Chicken Skeletal α-Actin Gene

The nucleic acid sequence of the skeletal α-actin gene has been characterized in chicken, rat, mouse and human. Fornwald et al, 1982, Nucl. Acids Res. 10:3861-3876; R. Zakut, 1982, Nature 298:857-859; French et al, 1990, Gene(Amst.) 88:173-180; Hu et al, 1986, Mol. Cell. Biol. 6:15-25; Minty et al, 1986, *Mol*. *Cell. Biol.* 6:2137-2148. The skeletal α-actin gene is a member of the actin multigene family, which, in vertebrates, is made up of three distinct classes of actin isoforms termed as "cytoplasmic", "smooth muscle", and "striated" on the basis of their cellular distribution and pattern of expression in adult tissues. The striated actins, α-cardiac and α-skeletal, are co-expressed specifically in cardiac myocytes and skeletal myofibers. Expression of the α-cardiac and α-skeletal actin genes is sequentially up-regulated in developing cardiac and skeletal muscle with the skeletal isoform predominating in adult skeletal muscle. (Vandekerckhove & Weber, 1984, *J. Mol. Biol*. 179:391-413; McHugh et al., 1991, *Dev*. *Biol*. 148:442-458; Hayward & Schwartz, 1986, *J. Cell Biol*. 102:1485-1493.) The chicken skeletal α-actin gene is the most highly expressed gene in adult chicken skeletal muscle comprising approximately 8% of the poly(A) RNA.

Numerous experiments *in vitro* and *in vivo* have established that the regulatory sequences which confer cell type restricted and developmentally regulated expression to the skeletal α-actin gene are primarily concentrated in the immediate 5' promoter region. (Bergsma et al., 1986, *Mol. Cell. Biol*. 6: 2462-2475; Taylor et al., 1988, *Genomics.* 3(4): 323-36; Petropoulos et al., 1989, *Mol. Cell. Biol.* 9:3785-3792; Carson et al., 1995, *Am. J. Physiol.* 268:C918-24.)

These regulatory sequences are highly conserved in the promoter regions of all of the known vertebrate skeletal α-actin genes from aves to man. Regulatory sequences derived from the chicken skeletal α-actin gene were utilized in construction of the IGF-I expression cassette, though other embodiments can utilize other actin or α-skeletal actin genes.

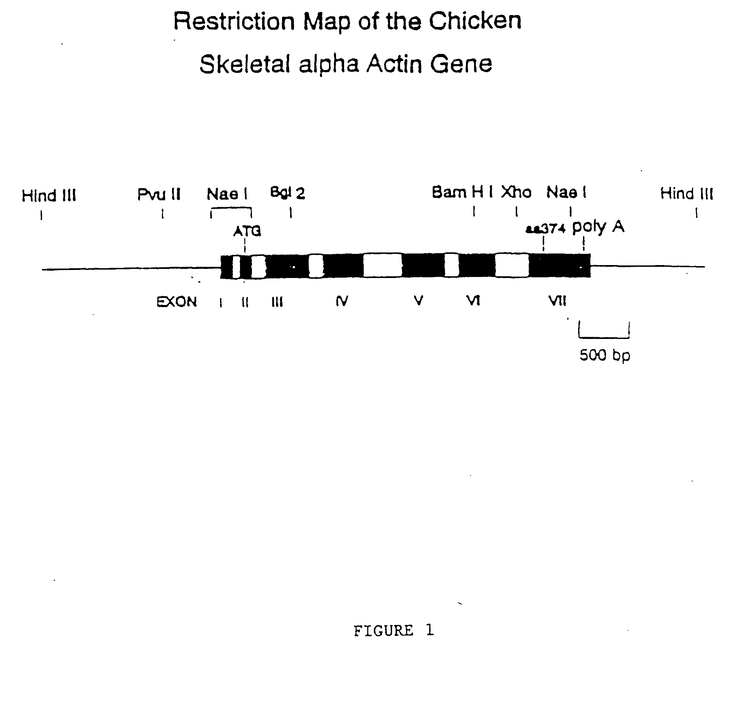

The primary sequences of the skeletal α-actin genes of the various species were deduced from overlapping cDNA clones. To obtain full genes, the cDNA clones were used to screen genomic DNA. For example, the 25 Kb EcoRI fragment of chicken genomic DNA isolated from a lambda Charon 4A vector, contains the 6.2 Kb skeletal α-actin gene on a single Hind*III* site of pBR322 is shown in Figure 1. Chang et al., *Mol. Cell. Biol.* 4:2498-2508 (1984). Nuclear transcription runoffs were used to map the transcriptional domain of the skeletal α-actin gene. The chicken skeletal α-actin control sequences have also been characterized (Bergsma et al., 1986, *Mol*. *Cell. Biol*. 6:2462-2475). DNA probes which encompassed portions of the 5' noncoding, promoter coding, and the contiguous 3' noncoding regions were cloned into M13 vectors which provided sense and antisense probes. Nuclei isolated from fibroblasts, myoblasts and day 19 embryonic muscle cells were used in *in vitro* transcription assays to extend RNA transcripts with radioactive tagged nucleotides. Labeled RNA hybridized to dotted DNA probes showed that transcription terminates approximately 1 kb downstream of the skeletal α-actin gene's poly A addition site. This is within a 800 bp Pvu*II* fragment between +2800 and +3600 nucleotides from the start of transcription.

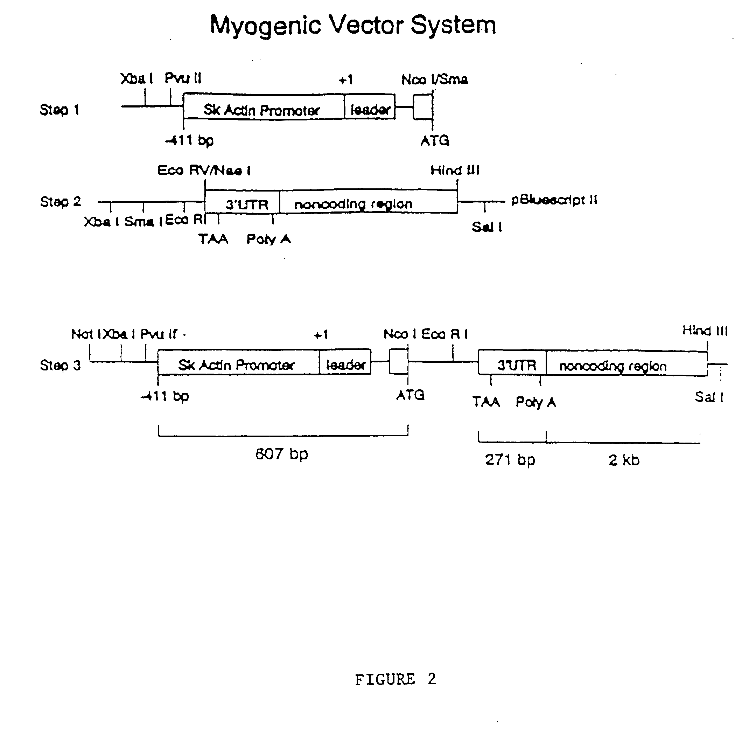

The 3' UTR and/or 3' NCR can be isolated by restriction endonuclease digestion of the 6.2 Kb actin gene with blunt cutter NaeI, which cuts 30 bp upstream of the translation termination codon TAA. Hind*III* releases the 3' most portion of the actin gene from the vector pBR322 (Figure 2). The 3'UTR and 3'NCR were used to prepare DNA constructs. The skeletal α-actin promoter and DNA flanking sequences (at least 411 nucleotides from the mRNA cap site) and DNA sequences extending through the skeletal 5' noncoding leader, first intron and up to the initiation of translation ATG, converted to a Nco*I* cloning site at +196, was liberated from a M13 double stranded DNA by Xba*I* and Nco*I* digestion, Klenow filled in and then linked into the XbaI and blunt SmaI sites of pBluescript II KS. The NcoI site is regenerated by this cloning step.

For certain vectors described in Schwartz et al., Application No. 08/472,809, the 3'UTR and 3'NCR on the 2.3 kb NaeI/HindIII fragment were directionally cloned into a blunt EcoRV site and the adjacent HindIII site of the pBluescript II KS vector cassette. The EcoRV and NaeI sites are destroyed. The restored NcoI site was used to insert cDNA sequences encoding polypeptides. Another cloning vector was constructed by inserting the skeletal α-actin promoter from -411 to -11 adjacent to the 3'UTR and 3'NCR. This expression vector eliminates the first intron and the skeletal actin 5' leader sequence. These two vectors were used in preparing DNA constructs to test the efficacy of the 3'UTR and 3' NCR.

Results obtained using vectors having a skeletal α-actin/IGF-I/skeletal α-actin expression cassette are described below, illustrating the intracellular expression of IGF-I from vector constructs and certain results of such expression.

For the exemplary vectors of the present invention, sequences including the skeletal α-actin promoter and first intron were utilized in conjunction with a IFG-I coding sequence and a hGH 3' UTR/poly(A) signal. Further results are presented below showing effects of IFG-I expression and certain comparative results with skeletal α-actin/IGF-I/skeletal α-actin containing vectors.

### Expression Vector Construction Containing Human IGF-I Gene

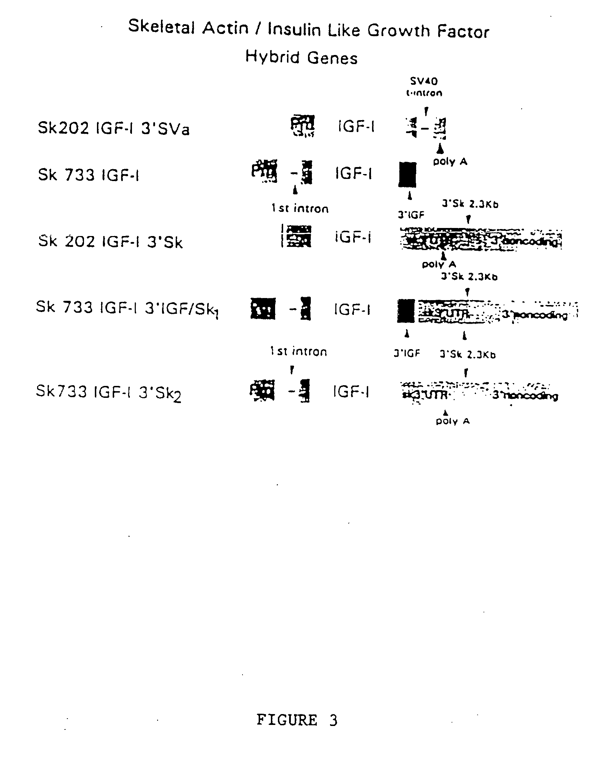

Constructions containing the skeletal α-actin promoter were linked to the human IGF-I cDNA (SEQ ID NO. 1) by standard recombinant DNA techniques as known in the art. Examples of a generalized expression vector structure utilizing skeletal α-actin 5' and 3' sequences is shown in Figure 2. Certain specific vector constructs with IGF-I are shown in Figure 3.

A first construction (SK202 SVa) was made so that the SV40 poly A addition site and the small t-intron were linked to the 3'UTR of the IGF-I cDNA. The SV40 sequences were added to increase the stability of nuclear IGF-I RNA transcripts. Since the SV40 t-intron might not be entirely suitable in the expression of IGF-I in muscle cells, five other vectors were made.

The SK733 NcoI vector contains approximately 411 nucleotides of the skeletal α-actin promoter, the natural cap site, 5' untranslated leader and the first intron. An NcoI site was engineered to create a unique insertion cloning site for the cassette containing the IGF-I cDNA, in which the initiation ATG was also converted to an NcoI site.

The SK733IGF-I construction utilizes its own poly A site. An NaeI/HindIII fragment which incorporated the skeletal α-actin 3' UTR, poly A addition site, and terminating sequences was linked to SK202, SK733 NcoI, IGF-I and to SK733IGF-I which the IGF-I poly A site was deleted and replaced by that of skeletal α-actin. In this way IGF-I RNA transcripts containing the skeletal α-actin 3' UTR are stabilized and accumulate in skeletal muscle cells. In addition, by providing contiguous 3' NCR, IGF-I is buffered against outside genomic sequences and is thus more protected from position effects, when integrated into the genome. In addition, by providing natural terminating sequences, the additional regulatory sequences that mark the transcriptional domain of skeletal α-actin prevent read through transcription, improve tissue specificity, developmental timing and transcriptional activity. Presence of 3'NCR sequence allows for a single copy of the integrated vector to produce 40-100% of the transcriptional activity of the endogenous sequences.

The SK733 IGF-ISK2 plasmid construct (pIG0100A) is disclosed in the Schwartz et al. application referenced above, Application No. 08/472,809. This plasmid has an ampicillin resistance backbone and encodes for IGF-I. The plasmid construct pIG0335 is similar to pIG0100A but it contains a Kanamycin resistance backbone, and is also disclosed in Schwartz et al., Application No. 08/472,809.

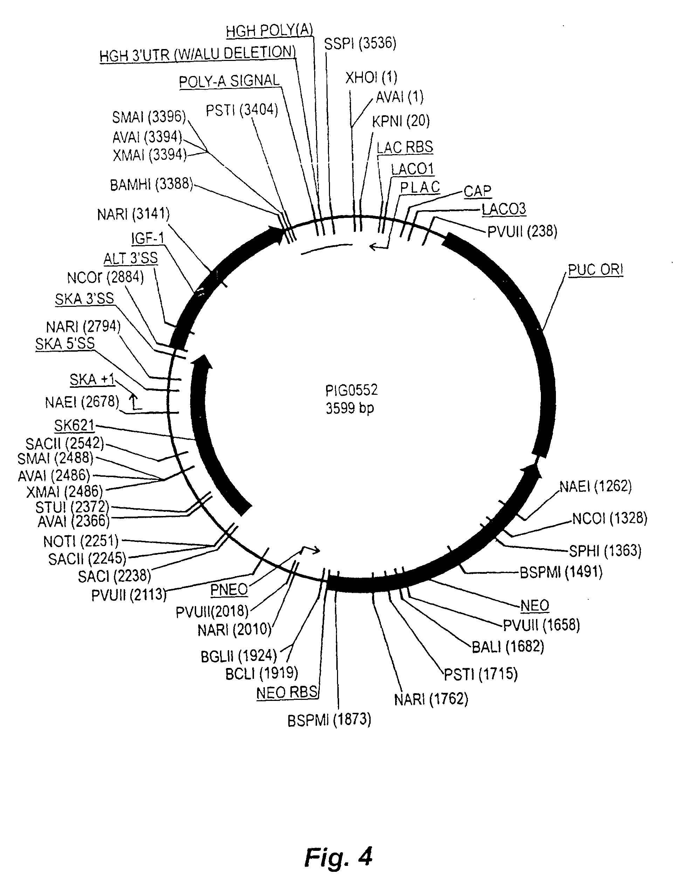

The exemplary plasmid vector, pIG0552 was constructed using pIG0100A and pIG0335B and additional constructs (pIG0376A and pVC0289A). A schematic representation of pIG0552 is shown in Fig. 4. The pIG0552B expression plasmid contains a hIGF-I gene expression cassette (Fig. 5) in a plasmid backbone containing a kanamycin-resistance (KanR) gene. The hIGF-I gene expression cassette of pIG0552B contains: 1) a promoter derived from the chicken skeletal α-actin promoter and first intron, 2) the human Insulin-like Growth Factor I (hIGF-I) cDNA, and 3) a 3' UTR/poly(A) signal from the human Growth Hormone (hGH) 3' untranslated region (3' UTR). The plasmid backbone is derived from pBluescript KS+ (Stratagene) with 1) the substitution of a kanamycin-resistance gene *(neo)* and prokaryotic promoter (pNEO, Pharmacia) in place of the ampicillin-resistance gene *(bla)* and 2) the deletion of the fl origin of replication.

Thus, the expression cassette described above differs from the original pIG0100 expression system specifically in the 3' UTR (pIG0100 contains skeletal actin 3' UTR; pIG0552 contains hGH 3' UTR). The hGH 3' UTR was substituted for the skeletal actin 3' UTR because it results in increased delivery of recombinant protein from skeletal muscle to systemic circulation. This result has been observed in both transgenic animal and non-viral gene therapy paradigms (i.e., both integrated and episomal template).

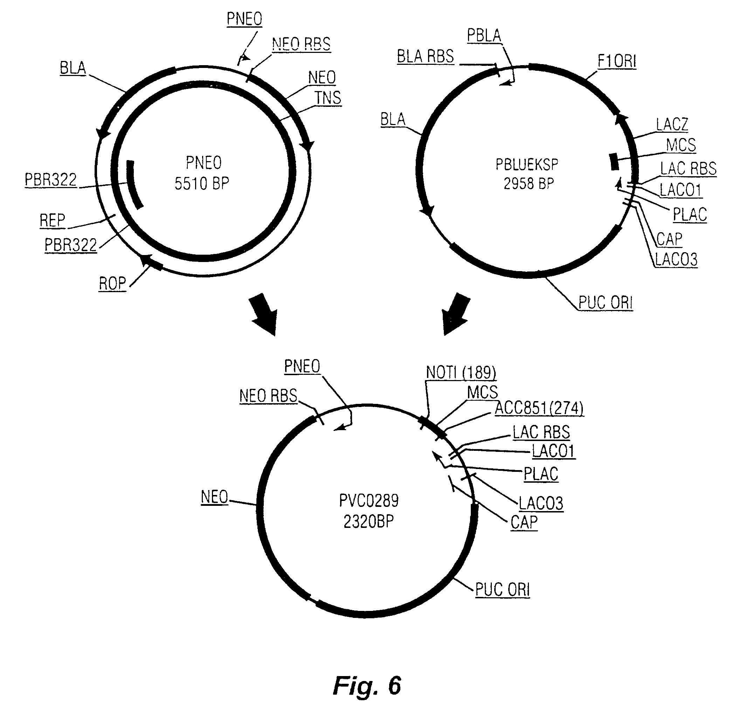

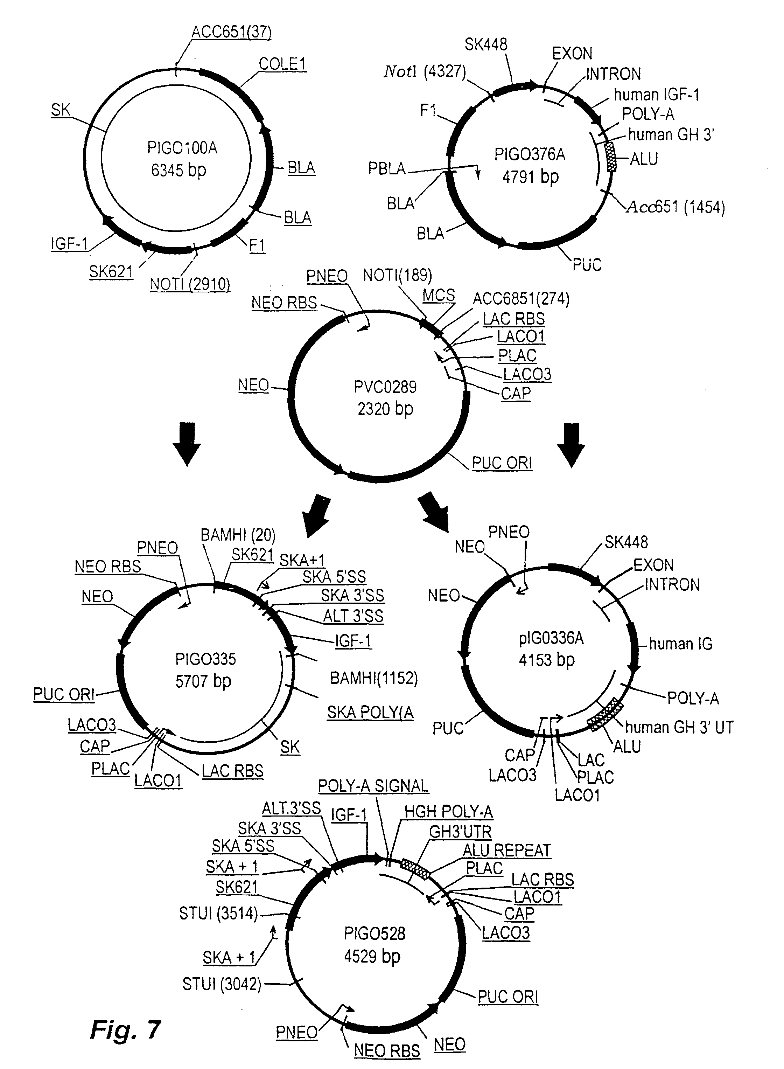

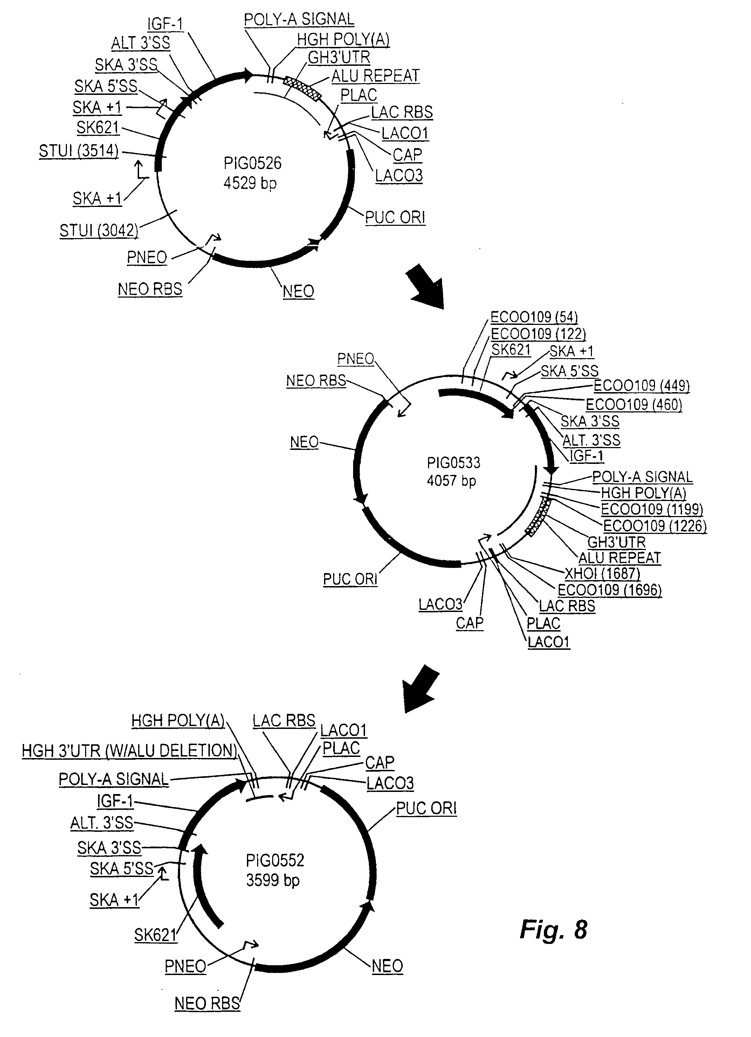

The actual construction of pIG0552B primarily involved three starting plasmids, pIG0100A, pIG0376A and pVC0289A. The process is shown schematically in Figs. 6, 7 and 8.

The chicken skeletal α-actin promoter and first intron and hIGF-1 cDNA were obtained from plasmid pIG0100A (R. Schwartz, Baylor College of Medicine). The hGH 3' UTR was obtained from plasmid pIG0376A (R. Schwartz, Baylor College of Medicine). pIG0100A contains the chicken skeletal α-actin promoter and first intron, human hIGF-1 cDNA, and chicken skeletal α-actin 3' untranslated region and 3' flanking sequence in pBluescript KS+. pIG0376A contains the chicken skeletal α-actin promoter and first intron, hGH leader sequence, hIGF-I cDNA, and hGH 3' UTR in pBluescript KS+. As indicated above, the plasmid backbone, pVC0289A, includes the kanamycin-resistance gene, pUC origin of replication, and a multicloning site.

The construction scheme used to produce pIG0552B from pIG0100A, pIG0376A, and pVC0289A required the construction of several intermediate plasmids. The first step in the construction of pIG0552B involved the transfer of the gene expression cassettes from pIG0100A and pIG0376A into pVC0289A, to produce pIG0335B and pIG0336A, respectively. pIG0335B was made by ligating the 3472 base pair (bp) NotI/Acc65I fragment containing the chicken skeletal α-actin promoter and first intron, hIGF-I cDNA, and chicken skeletal α-actin 3' UTR from pIG0100A into the NotI/Acc65I sites of pVC0289A. pIG0336C was made by ligating the 1918 bp NotI/Acc65I fragment containing the chicken skeletal α-actin promoter and first intron, hGH leader sequence, human IGF-I cDNA, and hGH 3' UTR from pIG0376C into the NotI/Acc65I sites of pVC0289A. pIG0526A was constructed by ligating the 1132 bp BamHI fragment containing the chicken skeletal α-actin promoter and first intron and the hIGF-I cDNA from pIG0335B to the 3397 bp BamHI fragment containing the hGH 3' UTR in kanR backbone and a fragment of chicken skeletal α-actin promoter. pIG0526A contains a duplicated portion of the chicken skeletal α-actin promoter. To delete the duplicated portion of the chicken skeletal α-actin promoter, pIG0526A was digested with StuI and the 4057 bp fragment containing the chicken skeletal α-actin promoter and first intron, hIGF-I cDNA, and hGH 3' UTR in the KanR backbone was religated, creating pIG0533A.

pIG0533A contains a human ALU repeat sequence downstream of the hGH 3' UTR. The human ALU repeat sequence in pIG0533A was deleted to create plasmid pIG0552B. The 395 bp EcoO1091 (blunt-ended with T4 DNA polymerase)/BspEI fragment containing the 3' portion of the hIGF-I cDNA and hGH 3'UTR excluding the ALU repeat from pIG0533A was ligated to the 3175 bp XhoI (bluntended)/BspI fragment containing the KanR backbone, chicken skeletal α-actin promoter and first intron, and 5' portion of the hIGF-I cDNA from pIG0533A to produce the final plasmid, pIG0552B. The deletion of the ALU repeat greatly reduces the frequency of integration of the vector into a human chromosome. However, both pIG0552 and pIG0533 were found to produce approximately the same amounts of secreted IGF-I.

The actual nucleotide sequence of plasmid pIG0552 was determined by standard methods. The expected nucleotide sequence was assembled electronically using Vector NT version 1.2 (InforMax, Inc., Gaithersburg, MD) from previously determined sub-sequences or retrieved from GenBank as follows: (1) the plasmid backbone which is a derivative of pBluescript (Stratagene) in which the *bla* (Amp^{r}) gene has been replaced with the neo (Kan^{r}) gene from transposon tn5 (nucleotides 1 - 2261); (2) skeletal α-actin promoter (nucleotides 2262 - 2688); (3) skeletal α-actin 5' untranslated region (UTR) and first intron (nucleotides 2689 - 2884) ; (4) human IGF-I coding sequence and a portion of the hIGF-I 3' UTR (nucleotides 2885 - 3392); (5) pBluescript multiple cloning site (MCS, nucleotides 3393 - 3409), and (6) human growth hormone 3' UTR (nucleotides 3410 - 3509) and 3' flanking sequence (nucleotides 3510 - 3600; GenBank accession #J03071, HUMGHCSA). The expected and actual nucleotide sequences for pIG0552 are shown aligned in Table I below.

The first base of the plasmid backbone sequence is arbitrarily designated nucleotide #1. Sequence identities between the aligned sequences are indicated by "|". Selected sequence elements are labeled and underlined for reference. Although only one strand for each sequence is depicted, over 47% (nucleotides 1884 - 3599) of pIG0552 was sequenced with multiple reads on both strands. Nucleotides 2268 through 3599 of pIG0552 were identical to the expected sequence. This region of the plasmid includes virtually all of the skeletal α-actin promoter and 5' UTR, the entire hIGF-I coding sequence (bolded), the hGH 3' UTR and flanking sequence. This confirms that this plasmid encodes a protein whose primary amino acid sequence matches that of the native human IGF-I protein.

A total of 8 nucleotide differences (indicated by "*") in other regions of the plasmid were observed between the actual and expected sequences. There is a single nucleotide deletion at position 21 in the expected sequence. This position is one base downstream from a Kpn I restriction site that is the last site in what remains of the pBluescript MCS. There is a single nucleotide difference at position 915 in the expected sequence. This position is in a non-critical region of the bacterial origin of replication. Finally, there are 6 nucleotide differences between positions 2262 and 2268 in the expected sequence. These positions are located at the cloning junction between the pBluescript MCS and the 5' end of the skeletal α-actin promoter sequence. The differences in this non-critical region are most likely the result of cloning artifacts. There is no evidence that any of the observed differences affect the relevant biological properties of pIG0552.

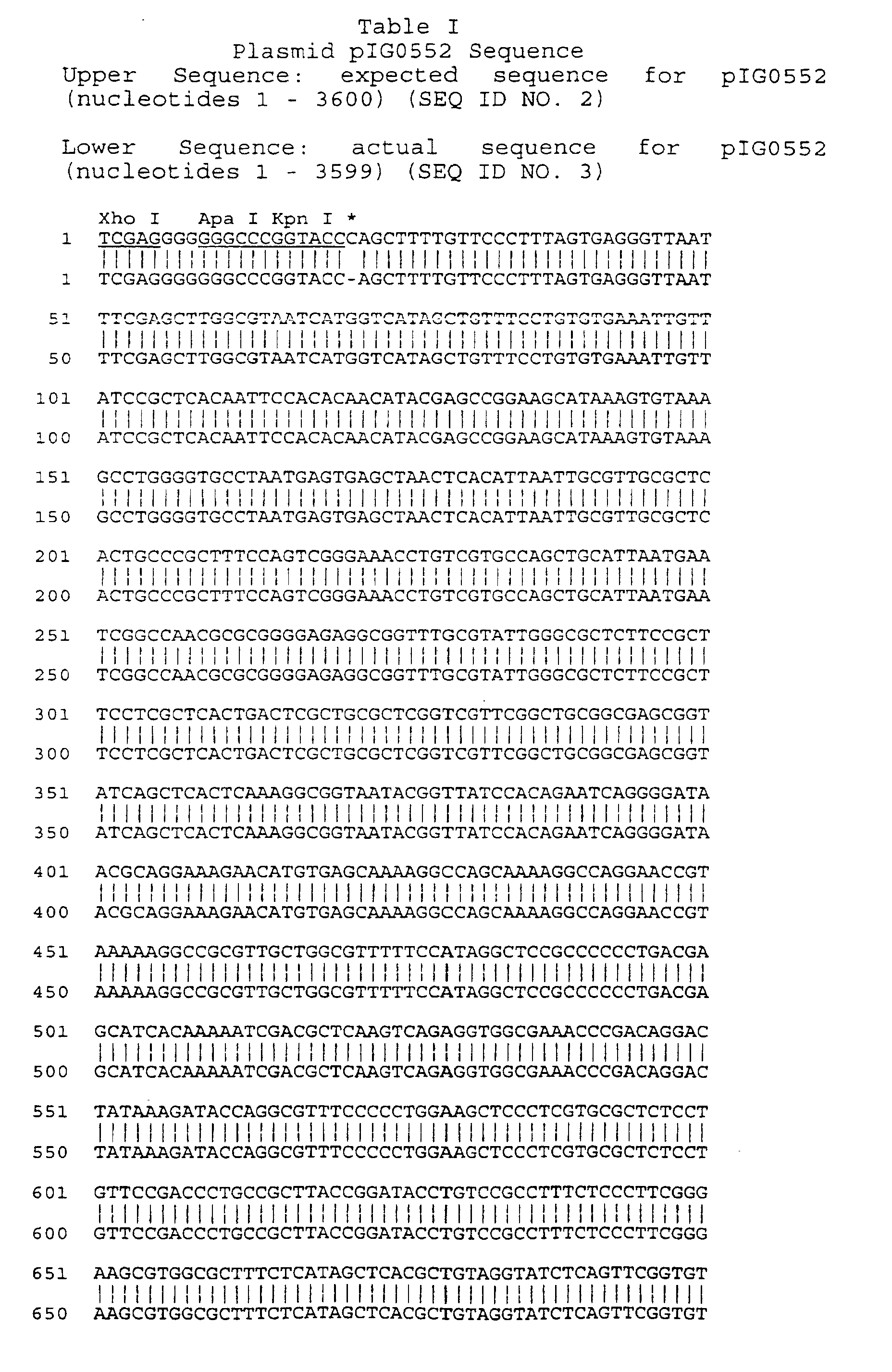

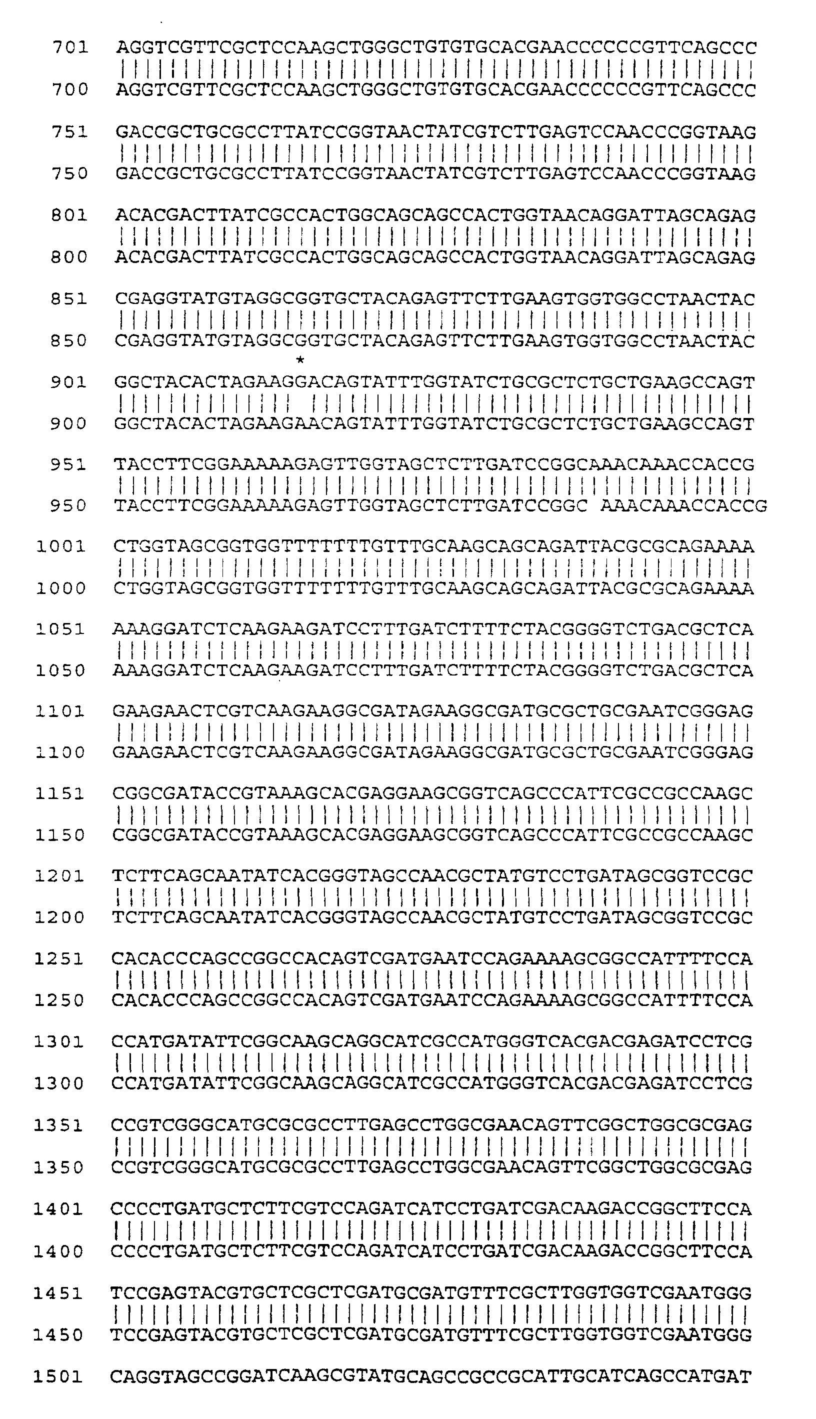

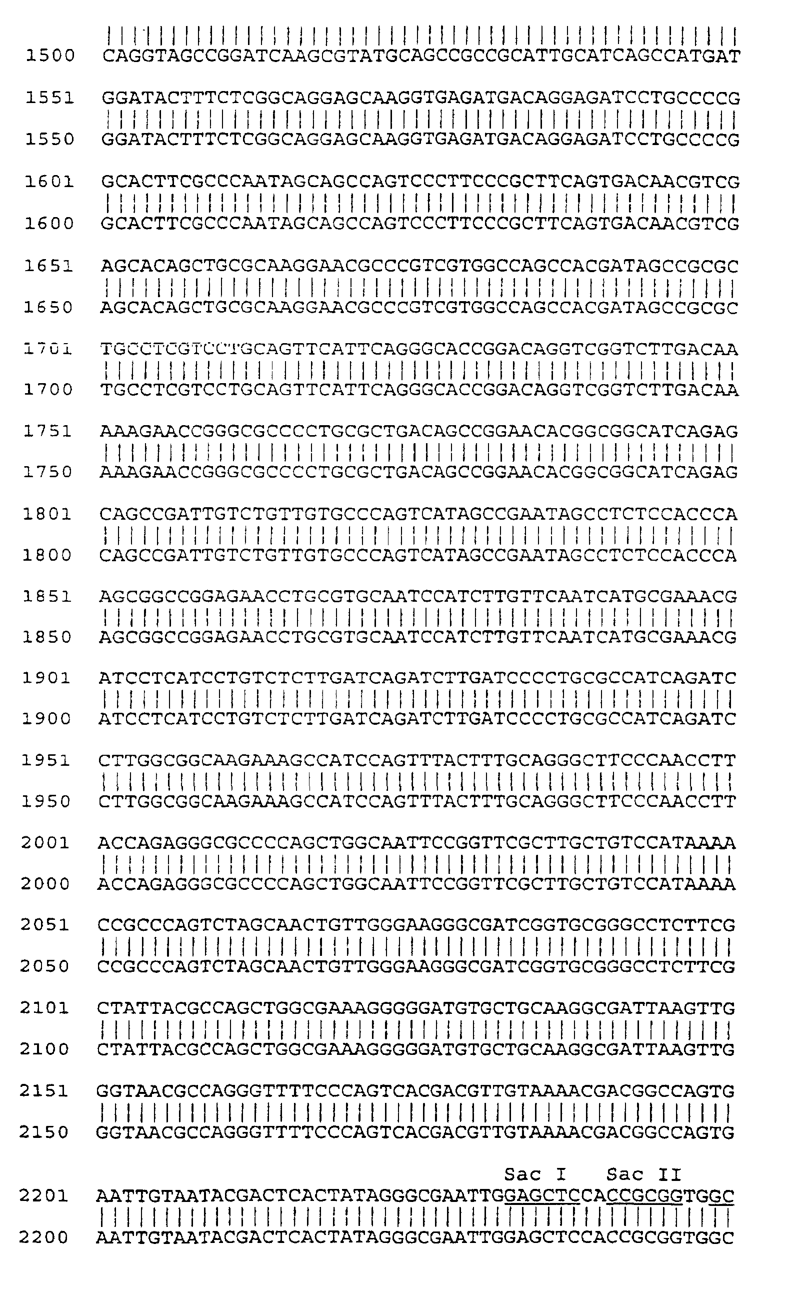

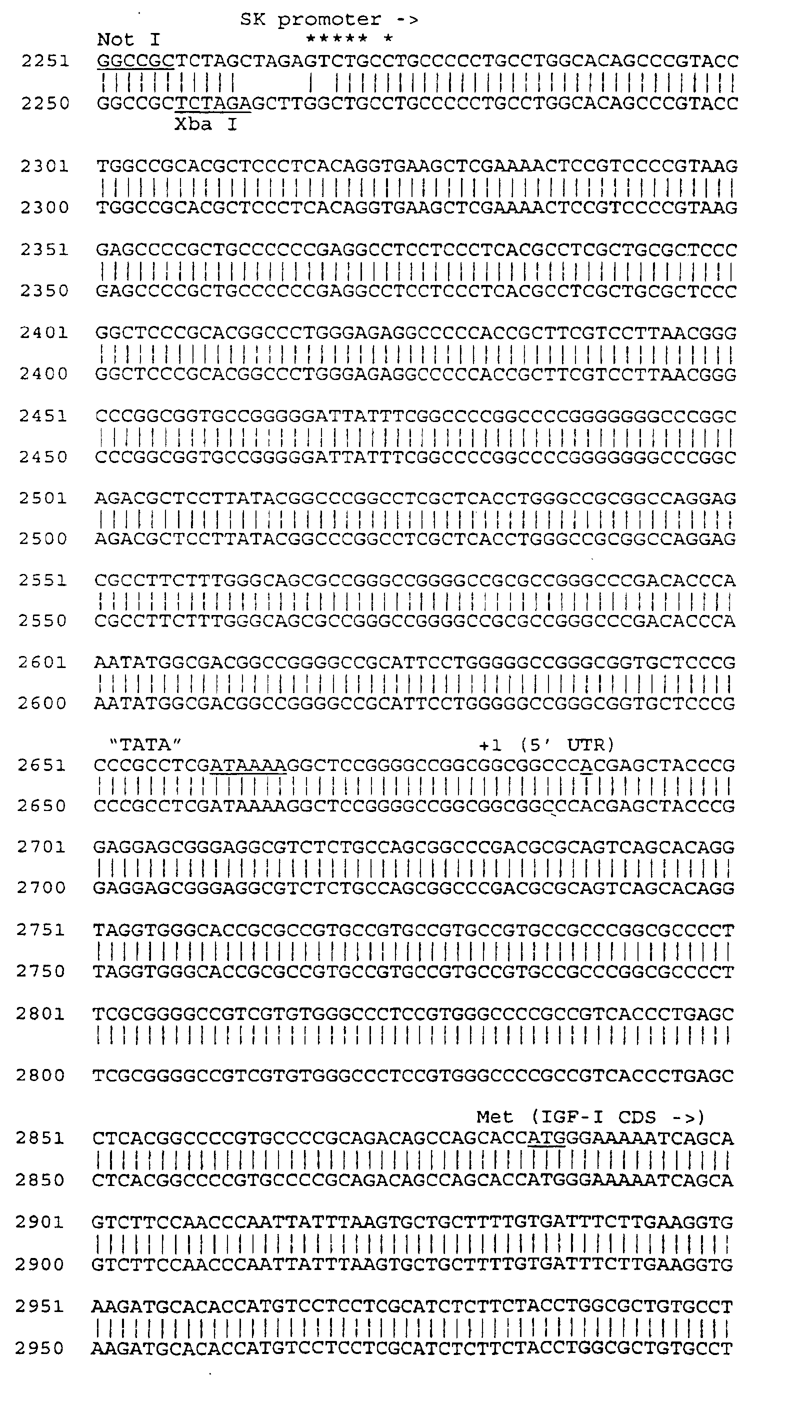

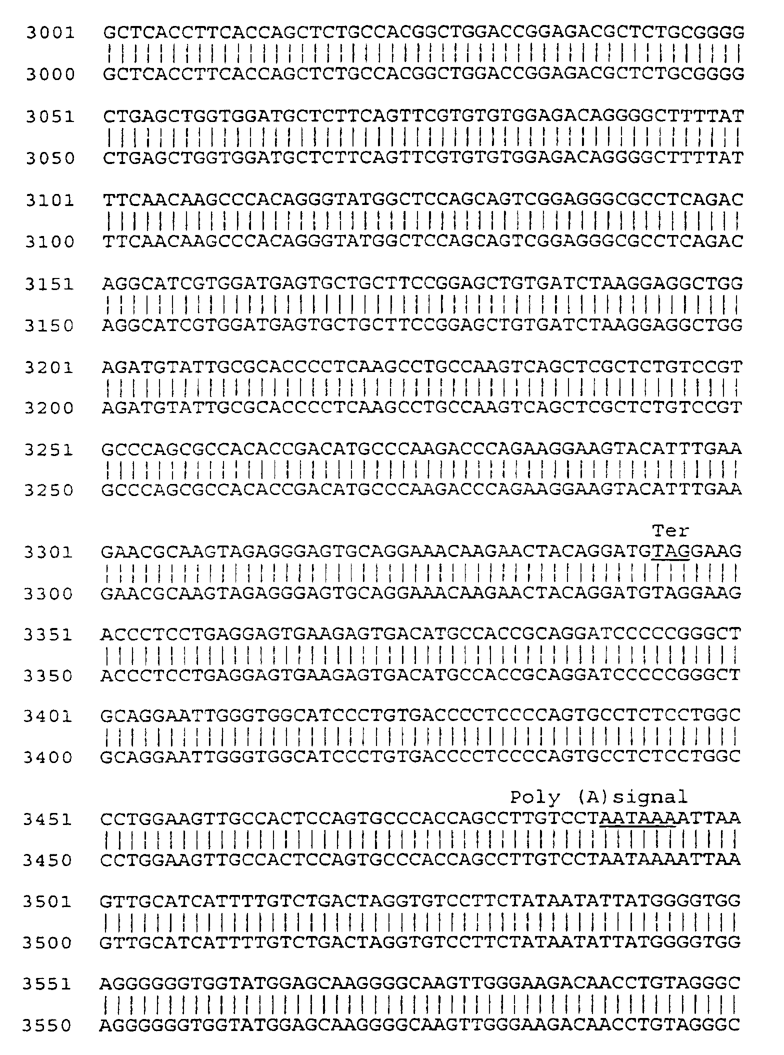

As noted above, evaluation of the exact sequence of pIG0552 demonstrated that a small number of sequence changes had occurred as compared to the resulting sequence predicted based on the sequences of the sequence components utilized. It was found that these changes did not occur in critical sequences. The presence of such changes in highly functional vectors provides further confirmation that vectors can incorporate a variety of different sequences while utilizing the same major sequence elements. Thus, the sequence disclosed is only exemplary.

Instead of the natural sequence coding for IGF-I, it is advantageous to utilize synthetic sequences which encode IGF-I. Such synthetic sequences have alternate codon usage from the natural sequence, and thus have dramatically different nucleotide sequences from the natural sequence. In particular, synthetic sequences can be used which have codon usage at least partially optimized for expression in a human. The natural sequences do not have such optimal codon usage. Preferably, substantially all the codons are optimized.

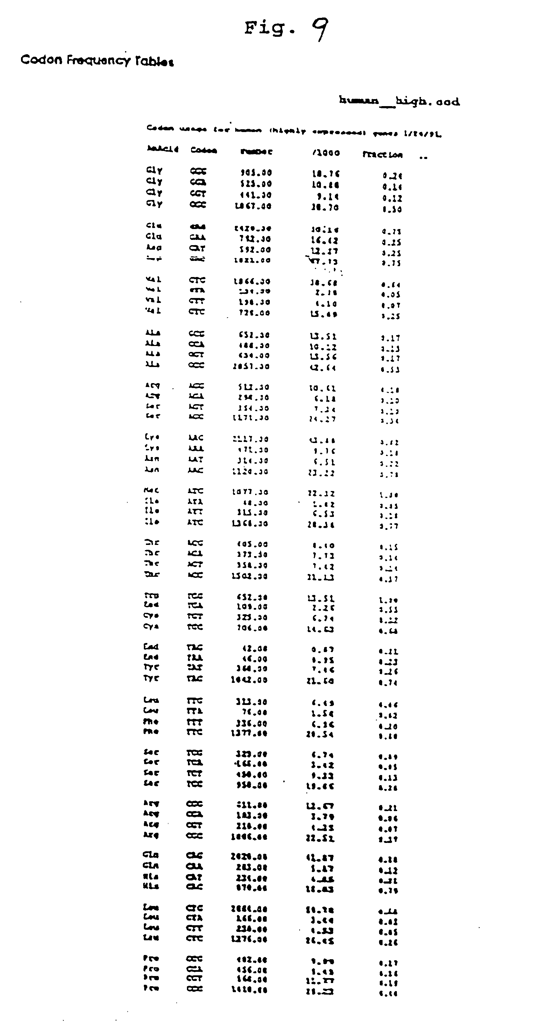

Optimal codon usage in humans is indicated by codon usage frequencies for highly expressed human genes, as shown in Fig. 9. The codon usage chart is from the program "Human_High.cod" from the Wisconsin Sequence Analysis Package, Version 8.1, Genetics Computer Group, Madison, WI. The codons which are most frequently used in highly expressed human genes are presumptively the optimal codons for expression in human host cells, and thus form the basis for constructing a synthetic coding sequence.

However, rather than a sequence having fully optimized codon usage, it may be desirable to utilize an IGF-I encoding sequence which has optimized codon usage except in areas where the same amino acid is too close together or abundant to make uniform codon usage optimal.

In addition, other synthetic sequences can be used which have substantial portions of the codon usage optimized, for example, with at least 50%, 70%, 80% or 90% optimized codons. Other particular synthetic sequences for IGF-I can be selected by reference to the codon usage chart in Fig. 9. A sequence is selected by choosing a codon for each of the amino acids of the polypeptide sequences. DNA molecules corresponding to each of the polypeptides can then by constructed by routine chemical synthesis methods. For example, shorter oligonucleotides can be synthesized, and then ligated in the appropriate relationships to construct the full-length coding sequences.

A particular preferred synthetic IGF-I coding sequence is provided in SEQ ID NO. 4.

### Preparation and Purification of IGF-I Plasmid

### A. Preparation of the master cell bank

Competant cells were transfected with the IGF-I plasmid pIG0552 described above. The cells utilized for tranformation were MAX Efficiency DH5∝™ Competent Cells (GIBCO BRL/Life Technologies). The Certificate of Analysis supplied with the cells shows that they exhibit a Lac⁻ phenotype (conferred by lac operon deletion), are inhibited by nitrofurantoin (demostrates *rec*A1 genotype), and are sensitive to antibiotics commonly used for plasmid stability (ampicillin, kanamycin and tetracycline). The published genotype of *E. coli* DH5∝ is F⁻φ80d*lac*ZΔM15 Δ (*lac*ZYA-argF)U169 *end*A1 *rec*A1 *hsd*R17 (r_{K}⁻m_{K}⁺) *deoR thi*-1 supE44 λ⁻*gyr*A96 *rel*A1.

Prior to the creation of the master cell bank (MCB), two lots of pIG0552 DNA were produced (pIG0552B.16S and pIG0552B.100). Due to the low plasmid yields and long fermentation time, clone selection was included in the development of the MCB.

To begin clone selection, three colonies were picked from a fresh transformation plate, and designated clones X, Y, and Z. These colonies were simultaneously streaked onto LB-Kan agar plates and inoculated into 50 ml LB-Kan liquid medium. As a control, 20 µl of pIG0552B was also inoculated into 50 mL LB-Kan; this was designated clone B. The agar plates were incubated overnight at 37°C, wrapped with parafilm and stored at 4°C. The liquid cultures were shaken for 17 hours at 37°C, 250 rpm and then placed in an ice bath.

Each of the liquid cultures were measured for plasmid yield. Specific yields (mg/gDCW) were 5.1, <1, 2.8, and 6.2 for clones B, X, Y, and Z, respectively. Ethanol precipitations were performed on each of the undiluted cell lysates.

Clone Z showed improved yields; therefore, five isolated colonies from the pIG0552Z agar plate were picked and inoculated into 500 mL of LB-Kan liquid medium in a baffled Fernbach flask with a foam and cheesecloth stopper. The culture was incubated with shaking for 16 hours, 37°C, 300 rpm and then placed in an ice water bath for cooling. The optical density of the culture was 3.4. Sterile 50% glycerol was cooled on ice, and 120 mL was added to the culture. The culture remained on ice with stirring while approximately 1 mL was dispensed into prelabeled cryovials. Vials were transferred to the -20°C freezer. The next day, the vials were transferred to - 80°C and then to liquid nitrogen for long term storage the following week.

To test the yield of the MCB, one vial was thawed, and 50 µL was used to inoculate 50 mL of LB-Kan liquid medium. After growth for 16 hours at 37°C, 250 rpm, the culture was analyzed for plasmid yield. The specific yield (mg/g DCW) was 5.2, which is within the expected limits for cultures started from a vial rather than an agar plate.

### B. Bulk Preparation

Bulk hIGF-I plasmid is produced using batch fermentation with *Escherichia coli* (*E. coli,* DH5-α) as the host organism. The fermentation and subsequent recovery process steps are described below. For the description below, the process is described taking the following as a basis: 1 liter broth, density (A₆₀₀ₙₘ) of 83, 39.9 g/L dry cell weight (DCW), and 5 mg/g DCW specific yield of crude plasmid, measured at prepurification. These are approximations; actual quantities will vary depending on the productivity of the fermentation.

### 1. Solutions used in the process.

Buffers, Media and Solutions Notes:
1) All quantities without ranges are nominal only.
2) Quantities with ranges are limited to the range specified.
3) Many buffers containing Tris-HCl are prepared with Tris base, using HCl for pH adjustment.

Kanamycin Sulfate - 20 mg/mL Kanamycin sulfate

dissolved in WFI, then 0.2 micron filtered and stored in a -20°C freezer.

Primary Seed media (LB) - Tryptone 10 g/L, yeast extract 5 g/L, sodium chloride 10 g/L, water.

Secondary Seed Medium - Soytone 15 g/L, yeast extract 15 g/L, sodium chloride 10 g/L, water.

Fermentation media (two parts) - (1) Sterilized portion (90%): glycerol 50 ml/liter, yeast extract 50 g/L, MgSO₄•7H₂O 4-6 g/L, (NH₄)₂ SO₄ 6 g/L; (2) Filtered portion (10%): thiamine hydrochloride 0.15 g/L, vitamin solution (see below) 1000x 3mL/L, K₂HPO₄ 6 g/liter, KH₂PO₄ 3-5g/L, trace metals solution (see below) 1000x 1-2 mL/L, 0.4 mL/L antifoam and 0025 - 0.5 mg/Kanamycin. All concentrations indicated based on the total volume.

Trace metals solution 1000x - FeCl₃•6H₂O 100 g/L, in sterile wash for irrigation.

Vitamin solution 1000x - Riboflavin 0.42 g/L, pantothenic acid 5.40 g/L, niacin 6.1 g/L, pyridoxine 1.4 g/L, biotin 0.06 g/L, folic acid 0.04 g/L, sterile water for irrigation, wrap in aluminum foil and store at 2-8°C.

Sodium hydroxide - 4-5 M NaOH in deionized water, for adjustment of the fermentation pH.

Phosphoric acid - 20% v/v concentrated phosphoric acid in deionized water, for adjustment of the fermentation pH.

Wash/resuspension buffer - 50 mM Tris-HCl, 10 mM EDTA, pH 8.0

Lysis buffer - 200 mM NaOH, 1% sodium dodecyl sulfate (SDS)

Neutralization buffer - A ratio of 35:13:8.75 of 3 M KOAc (pH 5.5), 5 M NaCl, and 7.5 M NH₄OAc.

RNase (100x) stock solution - 5 mg/mL RNase A, 10mM Tris-HCl, 15mM NaCl.

Water for irrigation - WFI in bags, purchased from a qualified vendor
Q Conditioning buffer - 2 M NaCl, 20 mM Tris-HCl, pH 8.0 prepared with WFI
Q Equilibration buffer - 0.625 M NaCl, 10 mM Tris-HCl, 1mM EDTA, pH 8.0 prepared with WFI
Q Wash buffer - Same as Q equilibration
Q Elution buffer- 0.75 M NaCl, 10 mM Tris-HCl, 1mM EDTA, pH 8.0
Q Regeneration solutions (1, 2, and 3) - (1) Same as Q and DEAE conditioning buffer, (2) 1 M NaOH, and(3) 1 M Acetic acid
Alkaline hydrolysis solution - 0.1 N NaOH prepared with WFI
Alkaline hydrolysis neutralization solution - 0.1 N HCl prepared with WFI
DEAE Conditioning buffer - same as Q conditioning buffer
DEAE Equilibration buffer - 0.33 M NaCl, 20 mM Tris-HCl, pH 7.5 prepared with WFI to a conductivity of 33 1 mS/cm
DEAE Wash buffer - Same as DEAE equilibration
DEAE Elution buffer - 0.4 mM NaCl, 20 mM Tris-HCl, pH 7.5, conductivity 39± 1 mS/cm
DEAE Regeneration solutions (1, 2, and 3) - (1) Same as Q and DEAE conditioning buffer, (2) 0.1 M NaOH, and(3) 0.1 M HCl
HIC Conditioning solution - 3.0 M Ammonium sulfate, prepared with WFI
HIC Equilibration solution - 1.5 M Ammonium sulfate, prepared with WFI
HIC Wash solution - same as HIC equilibration solution
HIC Regeneration solutions (1, 2, and 3) - (1) WFI, (2) 70% (v/v) ethanol, (3) 0.1 N NaOH prepared with WFI Note: WFI is used twice (see flow diagram)
UF/DF diafiltration solutions (1-2) - (1) 1M NaCl prepared with WFI, (2) WFI
Final product dilution - WFI

### 2. Fermentation Process and Isolation

Media Preparation. Media for the seed step is prepared in pre-sterilized Pyrex containers in approximately 2 liter quantities and steam sterilized. The antibiotic is then added after filtering with a presterilized 0.2 micron filter. This sterile seed media is stored at 4°C until needed. Fermentation media is prepared immediately before use. The basal media is sterilized in situ and 0.2 micron filter-sterilized antibiotic is added to the fermentor by aseptic transfer.

Fermentation Process. The process is started with a seed vial from the master cell bank (MCB).

The two-stage seed process begins by preparing the seed culture in a biological safety hood. 10-25 mL of sterile seed media is added aseptically to a presterilized flask. Filter-sterilized antibiotic solution is added to the appropriate final concentration (20-100 µg/mL). The culture is then inoculated with ≥200 µL of bacterial culture from the MCB vial. The flask is covered and placed in an incubator shaker. The seed culture is incubated at 37°C with shaking at about 250 rpm for two to six hours to develop the inoculum for the next stage. The next seed stage has the same antibiotic concentration, a volume of 1-10% of that used for the fermentation step, and is incubated similarly for two to eight hours.

The fermentation media contains 25-100 µg/mL of filter-sterilized antibiotic, which is added aseptically after media sterilization, to select against plasmid loss.

Fermentation starts when the inoculum from the seed is aseptically transferred to the fermentor. The fermentation is supplemented with up to 100 µg/mL antibiotic during the process to maintain selective pressure as cell density increases. Fermentation continues until an increase in dissolved oxygen indicates nutrient depletion, at which time the agitation is decreased and the culture cooled. After the temperature decreases to below 15°C, isolation steps are initiated. After fermentation, a sample of the culture is taken for a crude plasmid yield analysis, which is used to prepare for purification steps later in the process.

Isolation. The fermentation culture is centrifuged. The bacterial cell pellet is scraped from the centrifuge bowl(s) and transferred to presterilized 450 mL polypropylene bottles or resealable polyethylene bags for resuspension and mixing. The cells are washed once with an equal volume of wash buffer, centrifuged again and either stored at 2° to 8°C for no longer than 24 hours or stored at -20°C until the time of use.

### D. Purification

Prepurification. The cells are gently resuspended with the same buffer used for washing cells with a quantity sufficient for a total volume of about 7-12 mL/g of wet cell weight (WCW). Resuspended cells are gently transferred to a larger bottle or vessel and about 7-12 mL of lysis solution are added per gram (WCW) of starting cells to rupture cells and to denature cellular protein and chromosomal DNA. After addition of the lysis solution, the contents are gently mixed and held at room temperature (20-25°C) for about five minutes. Ice-cold neutralization solution is added, about 11-19 mL/g WCW, reducing the pH and precipitating cellular nucleic acids, protein, and chaotropic agent from the lysis buffer. The resulting suspension is held while cooling for a minimum of 1/2 hour.

Buffers and solutions prepared for this and other steps are 0.2 micron filtered and stored either in presterilized pyrex bottles or in sterile and endotoxin-free disposable bags. The water used is sterile water for irrigation (WFI).

Solid-liquid separation is performed initially via centrifugation. Centrifugation is performed at 0-8°C. The supernatant, containing fine colloidal particles, is 0.3 micron-filtered to remove the remaining precipitate, completing the solid-liquid separation. The final container used is presterilized, washed again with 0.5N sodium hydroxide and then triple-rinsed with WFI. The same treatment is applied to all product containers and transfer tubing used after this point with the exception of the containers used for pure bulk storage.

RNaseA stock solution, 100x concentration is stored at -20°C. RNA is digested after equilibrating the filtered alkaline lysis pool to room temperature and adding 0.01 v/v of RNase stock solution. The solution is incubated for a minimum of sixty minutes at a temperature of 30-45°C. The resulting solution is processed by chromatography immediately, or held overnight at 2 to 8°C in sterilized Pyrex containers.

Purification. The material is filtered through a 0.2 micron filter prior to chromatography. The supernatant containing plasmid, other cellular nucleic acids and protein is diluted three-fold with two volumes of WFI.

In the Q anion exchange step, the resin (Pharmacia Q high performance) is treated with 1 N. NaOH for 30-35 minutes in the column as a precautionary measure. The column is conditioned with about 5 column volumes (CV) of Q and DEAE conditioning buffer, then equilibrated with about 5 CV of Q equilibration buffer (volume may be less if determined to be acceptable by pH and conductivity). The column feed rate is a linear velocity of about 155 cm/hr for all steps. The diluted feed is loaded to a maximum of one mg of crude plasmid per mL of resin. After the load, the column is washed with Q wash buffer for one additional CV after the column detector indicates output has leveled close to the baseline. The product is eluted with about 5 CV of elution buffer, with the actual peak on the chromatogram indicating when eluate collection starts and ends. The column is regenerated with about 5 CV each of the three Q regeneration solutions. The resin is stored in the column until the next use after pumping 5-10 CV of 0.01 N NaOH through the column, or cycled again. The Q eluate may be stored at 2 to 8°C.

The RNA is checked using a crude plasmid analysis. If the ratio of the front RNA-containing peak to the second products containing peak is above a pre-set limit, a contingency alkaline hydrolysis and neutralization procedure is performed. Then 0.1 N NaOH is slowly added to the Q eluate with gentle mixing to achieve a final pH of 11.2-11.3. The pH of a sample of the treated solution is measured and recorded. The solution is held for about ten minutes at 20-25°C. Afterwards, 0.1 N HCl is added by slow addition with gentle mixing to neutralize the solution; the grid pH (between 7.5-8.0) is measured and recorded.

The pool is diluted about two-fold with WFI to give an appropriate salt concentration for the second purification step. As a precautionary measure in the DEAE anion exchange step, the resin (Tosohaas DEAE 650S) is treated with 0.1 N NaOH for 30-35 minutes in the column. The column is conditioned with about 5 CV of Q and DEAE conditioning buffer, then equilibrated with about 5 CV volumes of DEAE equilibration buffer. The column flow rate is a linear velocity of about 155 cm/hr for all steps. The feed is loaded to a maximum of 0.7 mg of crude plasmid per mL of resin. After the load, the column is washed with DEAE wash buffer for one additional CV after the column detector indicates output has leveled close to baseline. The elution takes place with about 5 CV of DEAE elution buffer with the beginning and end of peak collection determined by the chromatogram. The column is regenerated with about 5 CV each of three regeneration solutions. The resin is stored until the next use after pumping 5-10 CV of 0.01 N NaOH through the column or cycled again. The DEAE eluate is stored at 2 to 8°C.

The DEAE eluate is diluted two-fold with hydrophobic interaction chromatography (HIC)-conditioning solution. The HIC resin (Tosohaas Phenyl 650S) is treated with 0.1 N NaOH for 30-35 minutes in the column, as a precautionary measure. The column is equilibrated with about 5 CV HIC of equilibration buffer. The column feed rate is a linear velocity of about 75 cm/hr for all steps. The feed is loaded to a maximum of 0.5 mg of crude plasmid per mL of resin and the flow through is collected. After the load, the column is washed with one CV of HIC equilibration buffer after the detector indicates the chromatogram is close to baseline; the wash is collected with the flow-through. The column is regenerated with about 5 - 10 CV of each of the three regeneration solutions. The resin is cycled again or stored until the next use after pumping 5-10 CV of 20% (v/v) ethanol through the column. The HIC eluate is stored at 2-8°C.

Alternatively, the purification process can be as described in U.S. Patent Application 60/022,157.

### Myogenic Cell Cultures

Primary chicken myoblast cultures from breast muscles of day 11 white leghorn chick embryos were developed according to the protocol described in the art. Grichnik et al., *Nucleic Acids Research* 14:1683-1701 (1986). Enriched myoblasts were plated at a density of 2 X 10⁵ cells per 60 mm collagenized tissue culture dish.

Myogenic mammalian C₂C₁₂ and Sol 8 cells (1 X 10⁵) were subcultured onto 60 mm dishes one day before transfection.

### DNA Transfer

Tissue culture cells were transfected with plasmid DNA by the calcium phosphate precipitation-glycerol shock protocol as known in the art. Wigler et al., Cell 14:725-731 (1978). A total of 10 µg of DNA was used to transfect each 60 mm dish of tissue culture cells. Transfections were done in quadruplicate and with three different MVS-CAT-MLC plasmid preparations to control for variations in DNA quality and plating density of cells.

### CAT Assay

After transfection two populations of cells, coinciding with replicating myoblasts and post-fusion myotubes were harvested, and assayed for CAT activity as described in the art. Gorman et al., *Molec*. *Cell. Biol.* 2:1044-1051 (1982). Cell pellets were lysed by repetitive freeze thaw cycles in 50 µl of 250 mM Tris-HCl ph 7.5. The production of acetylated [¹⁴C] chloramphenicol (0.5 µCi per assay, 57.8 mCi/mMol) was assayed for 90 minutes at 37° C. Acetylated chloramphenicol was monitored by autoradiography following thin layer chromatography on silica gel plates. Separated acetylated chloramphenicol spots were quantitated by scanning on a Betagen phosphoimager screen. Data was expressed as the percentage of converted [¹⁴C] chloramphenicol per µg cell protein. Protein concentration of cell extracts was determined by the method of Bradford, *Anal. Biochem.* 72:254-258 (1976)) at each time point to ensure uniformity in the assays.

### Splicing of IGF-I Constructs

As described above, the pIG0100 construct was cloned to include the chicken skeletal actin promoter and intron (including the 5' and 3' splice sites), the human IGF-I 48 amino acid signal peptide, the 70 amino acid mature protein and the E peptide. The RNA produced from this expression system does not use the actin 3' splice site, instead it splices to a site in the IGF-I signal peptide sequence. The splicing has been confirmed by sequencing of RT-PCR products. It is believed that the resulting polypeptide has a 25 amino acid signal sequence, a form which is naturally occurring in muscle and many other tissues. Adamo *et al*., 1994, *Adv. Exp. Med. Biol*. 343:1-11.

The pIG0552 construct contains the same upstream sequences as the pIG0100 construct with the human grwoth hormone 3' UTR instead of the chicken skeletal α-actin 3' UTR. It is believed that the splicing of the pIG0552 product is the same as for the pIG0100 product. It has been confirmed by agarose gel analysis of RT-PCR products that the products from both constructs are the same size.

### Activity of Expression Vector Constructs

To determine the efficacy of actin promoter/gene IGF-I hybrid genes in mouse myogenic cells the expression vector was studied using these genes in the background of mammalian C₂C₁₂ myoblasts by making a population of stable transfected C₂C₁₂ myoblasts. The altered IGF-I expression levels were directly evaluated in these stable myoblast cell lines. Each IGF-I construction shown in Figure 3 was co-transfected with the drug selectable vector EMSV-Hygromycin into mouse C₂C₁₂ cells. After two weeks of selection, a population of stable myoblasts was selected. A population of C₂C₁₂ myoblasts stably transfected only with EMSV-Hygromycin served as the controls. Visual inspection of the transfected myoblast revealed several insights into the role of IGF-I on muscle cell differentiation that would not be obvious in transgenic mice. In general all of the myogenic cell lines containing IGF-I genes caused myoblasts in growth media (10% fetal calf serum) to replicate more extensively than controls. Changing culture medium to 2% horse serum initiates the differentiation process. In the process, control C₂C₁₂ myoblasts fuse to form multinucleated myotubes over a period of four days. At the same cell density per culture dish, myoblasts containing SK733IGF-I, SK202IGF-I-SK, SK733IGF-I-SK1 and SK733IGF-I-SK2 fused at least two-to-three days earlier than C₂C₁₂ or EMSV-Hygromycin control myoblasts.

In order to study the steady state accumulation of IGF-I MRNA in C₂C₁₂ myoblasts, equal amounts of total cellular RNA was isolated from stably transfected C₂C₁₂ myoblasts grown in growth media ("G") or differentiation media ("D"). The RNA was electrophoretically separated on denaturing agarose gels, transferred onto nylon filters and probed with uniformly ³²p labeled full length human IGF-I cDNA under standard hybridization techniques. The intensity of the autoradiographic signal on X-ray film provides a relative measure of mRNA accumulation, an overall index of combined transcriptional activity and mRNA stability of the expression vectors. The IGF-I mRNA in vector, SK202IGF-I-3'SVa did not accumulate in myotubes above myoblast levels. This is a typical expression activity. The SK733IGF-I vector contains the IGF-I 3'UTR. The IGF-I mRNA from this vector accumulated in myotubes but at levels substantially lower than SK202IGF-I-SK or SK733IGFI-SK2. These latter two vectors contain the skeletal actin 3'UTR and 3'NCR. Since, the primary difference in these vectors is the 3'UTR, the increased stabilization of the RNA transcripts due to the skeletal 3'UTR accounts for about a 100-fold difference in RNA content.

In a similar assay, IGF-I was also produced at high levels from pIG0552 in C₂C₁₂ cells.

### Measurement of Secreted Levels of IGF-I from IGF-I Gene Delivery by the Expression Vector

In order to measure the amount of IGF-I synthesized and secreted into the media, differentiated myotube cultures were grown in minimal media (DMEM and 0.05% bovine serum albumin, RIA grade). SK733IGF-I-SK2 is the most effective construction to express IGF-I in muscle cells. IGF-I was assayed by both radioimmunoassays of tissue culture media and by immunoperoxidase staining of cells. Increased levels of IGF-I during the fusion of several muscle cultures was found. The comparison of levels from different expression vectors are shown in Table II. In control cultures, the level of IGF-I was in the range of 0.2-0.5 ng/ml. In comparison, vector SK733IGF-I-SK2 (pIG0100A or pIG0335) has levels of IGF-I at least one hundred times greater.

**Table II**

| IGF-I Levels in Stably Transfected C₂C₁₂ Mvoblasts | |
|---|---|
| Construction | IGF-I |
| (ng/ml of media/4 days) | |
| SK202IGF-I-3'SVa | 4.4 |
| SK733IGF-I | 3.8 |
| SK733IGF-I-SK2 | 79.0 |
| Control C₂C₁₂ | 0.5 |

In a similar manner, immunoperoxidase staining of myogenic cultures revealed the increased production of immunological reactive IGF-I in stable transfected myoblasts but not in the control EMSV-Hygromycin transfected myoblasts or in perfusion C₂C₁₂ cells. Antibodies against the A and D regions were used at dilutions of 1:1000. All of the transfected lines including SK202IGF-I were positively immunoperoxidase stained. Thus, it is clear that enhanced levels of IGF-I are being synthesized and exported from the stable myoblasts.

### Insertion of Expression Vectors into Transgenic Mice

Transgenic mice carrying hIGF-I containing vectors were generated by standard oocyte injection (Brinster, et al, *Proc. Natl. Acad. Sci. USA* 82:4438-4442 (1958)) and bred to demonstrate stable transmission of transgenes to subsequent generations. Transgenics were identified by polymerase chain reaction or Southern genomic DNA blotting analysis from tail cut DNA. Transgenics were tested for muscle specific expression of the transferred IGF-I vector by RNA blotting of total RNA isolated from several tissues. Independent transgenic mouse lines 5484, 5496, 5832, 5834 were generated with SK202IGF-I-3'SVa, containing the SV40 3' intron and poly A addition sequence. Mice from these strains were found to have weak expression, primarily in heart tissue, but very low levels were found in skeletal muscle and non-myogenic tissues such as the kidney and brain. Independent transgenic mouse lines 3357, 3359 generated with SK733IGF-I-3'SK2 (pIG0100A or pIG0335). Mice from these strains were found to have elevated expression levels of IGF-I. These levels are comparable to the endogenous mouse α-actin gene activity. These levels from SK733IGF-I-3'SK2 (pIG0100A or pIG0335) show at least 100-1000 fold greater accumulation of IGF-I mRNA in comparison to the levels produced by the SK202IGF-I-3'SVa vector. The addition of the skeletal α-actin 3'UTR and 3' flanking region allowed for a preferential increase in IGF-I RNA in skeletal muscle rather than cardiac. Thus, the 3'UTR and 3' NCR of skeletal α-actin enhance muscle specific gene expression.

Mice from these strains demonstrated increased muscle mass and reduced percentages of body fat as compared to the parental types. The use of human IGF-I in the mouse demonstrates the cross-species applicability of this particular gene.

In addition, by providing contiguous 3' NCR, IGF-I is buffered against outside genomic sequences and is thus more protected from position effects, when integrated into the genome. Also, by providing natural terminating sequences, the additional regulatory sequences that mark the transcriptional domain of skeletal a-actin prevent read through transcription, improve tissue specificity, developmental timing and transcriptional activity. Presence of 3'NCR sequence allows for a single copy of the integrated vector to produce 40-50% of the transcriptional activity of the endogenous sequences.

### Somatic Gene Transfer to Skeletal Muscle In Vivo

To demonstrate an effect of the IGF-I encoding vectors as used in in vivo gene therapy, vectors were injected into adult muscle for the express purpose of expression of a particular polypeptide. The growth hormone-deficient mouse strain, *little*, was used in these studies. Vector SK733IGF-I-SK2 (pIG0100A or pIG0335), or control vector SKSK, was pelleted by sedimentation, dried under vacuum and punctured into the quadricep muscle (20 µg/pellet - 3 pellets/muscle) of 2 sets of 6 *little* mice. The entire muscle from each animal that received an inoculation was removed 2 weeks following introduction of the DNA and assayed for IGF-I protein in the tissue. The amount of IGF-I in each tissue was assayed by using a radioisotopic assay. A slight yet significant (p>0.05) increase was observed in IGF-I expression (Table III) from 4.2 ng to 6.9 ng IGF-I/100 µg total protein of muscle lysate in mice with vector only (no IGF-I) for mice with the vector SK733IGF1-3'SK.

**TABLE III**

| IGF-I Levels in Tissues of IGF-I Vector-Injected little MICE | | | |
|---|---|---|---|
| Mouse# | Strain | Plasmid | IGF-I (ng/100ug) |
| 776 | *little* | PSKSK | 4.2 |
| 777 | *little* | PSKSK | 4.2 |
| 778 | *little* | PSKSK | 4.5 |
| 779 | *little* | PSKSK | 3.9 |
| 780 | *little* | PSKSK | 3.9 |
| 781 | *little* | PSKSK | 4.2 |
| Average | | | 4.15+0.21 |
| 782 | *little* | pSK733IGFSK | 4.5 |
| 783 | *little* | pSK733IGFSK | 6.3 |
| 784 | *little* | pSK733IGFSK | 8.2 |
| 785 | *little* | pSK733IGFSK | 6.9 |
| 786 | *little* | pSK733IGFSK | 8.4 |
| 787 | *little* | pSK733IGFSK | 7.0 |
| Average | | | 6.88±1.08 |

### Intramuscular Injections of a IGF-I Myogenic Vector in Diabetic Rats.

The effect of intramuscular injections of a muscle-specific DNA vector carrying the human insulin-like growth factor-I ("IGF-I") on diabetes-induced alterations in body and muscle weights, plasma glucose levels and the mRNA level from the injected IGF-I vector was examined. An IGF-I expressing vector was chosen for this work since injections of recombinant IGF-I have been shown to have anabolic effects in a number of models of cachexia.

Diabetes was induced in male Sprague-Dawley rats (175200 g) with intravenous injections of streptozotocin (STZ; 55 mg/kg) dissolved in sodium citrate buffer (0.05 M, pH 4.5). Control non-diabetic animals were age, weight and sex matched and received equal volume injections of vehicle. Diabetes was confirmed by the onset of hyperglycemia, glucosuria, and reduced rate of growth. Three days following STZ administration, non-fasted animals were anesthetized with pentobarbital (50 mg/kg) and blood samples were obtained by cardiac puncture. Blood was transferred to EDTA-containing tubes, centrifuged at 3000 x g for 15 min and stored at -70°C. The gastrocnemius was injected bilaterally following direct visualization of the muscle via a cutaneous incision. The right gastrocnemius muscle of individual rats was injected with either 0, 50, 200, or 800 µg of IGF-I vector in 200 µl of isotonic saline solution. The contralateral (left) gastrocnemius received 200 µl injections of isotonic saline. The IGF-I vector used in this series of experiments was Sk-733-IGF-I-Sk2 as described above. Six days following intramuscular injection of muscle-specific vector, the animals were deprived of food (12-16 hrs) followed by euthanization by decapitation. Blood was then collected and the entire gastrocnemius muscle was removed (dissection from tendon to tendon).

For the analysis of vector effects on body and muscle weight dosage groups were matched on pre-vector injection body weight and only diabetic animals were included in the analysis. The plasma glucose criteria for inclusion in the analysis was a non-fasting plasma glucose level greater than 300 mg/100 ml. Pre-vector injection body weights were matched by only including animals with body weights between 175-195 gm. For the analysis of vector effects on plasma glucose levels the groups were matched on pre-vector injection plasma glucose levels. Intramuscular injections of IGF-I vector result in increased body weight (Mean ± SD; Vehicle Only = 181.37 ± 6.17; 50 µg = 193.43 ± 5.71; 200 µg = 186.6 ± 8.01; 800 µg = 191.14 ± 7.54). This body weight increase is statistically significant at the 50 and 800 µg, but not the 200 µg, dose level (a priori t-test: Control vs. 50 µg, t = 3.57, df = 12; Control vs. 200 µg, t = 1.17, df 10; Control vs. 800 µg, t = 2.29, df = 12).

In addition to increasing body weight IGF-I vector injections also increase the weight of the vector injected gastrocnemius (Mean ± SD; Vehicle Only = 1.00 ± 0.08; 50 µg 1.10 ± 0.07; 200 µg = 1.07 ± 0.03; 800 µg = 1.09 ± 0.05) This increase in vector injected gastrocnemius weight is statistically significant at the 50 and 800 µg, but not the 200 µg, dose level (a priori t-test: Control vs. 50 µg, t = 2.32, df = 12; Control vs. 200 µg, t = 1.75, df 10; Control vs. 800 µg, t = 2.32, df = 12). The weight of the contralateral gastrocnemius was also increased but this increase did not reach statistical significant (Mean ± SD; Vehicle Only = 1.00 ± 0.07; 50 µg = 1.07 ± 0.06; 200 µg = 1.05 ± 0.01; 800 µg = 1.08 ± 0.06; a priori t-test: Control vs. 50 µg, t = 1.72, df = 12; Control vs. 200 µg, t = 1.43, df 10; Control vs. 800 µg, t = 2.11, df = 12).

The level of expression of the injected IGF-I construct was assessed by determining the level of IGF-I specific mRNA. Whole cell RNA isolated from the injected and control, contralateral, gastrocnemius, was treated with DNAase and subjected to reverse transcription using oligo-dT as a primer in order to generate cDNA replicas of mRNA. The cDNA was than reacted with IGF-I specific primers in a polymerase chain reaction to estimate the level of expression of mRNA in the original muscle sample. The bands corresponding to IGF-I-specific primer amplified products were detected. The data indicates that the IGF-I vector IGF-I construct is being expressed at significant levels in the injected muscle. The control muscle showed no expression of human IGF-I.

Relative to the Control group fasting plasma glucose levels in the 50 µg IGF-I vector dose group were significantly lower (Mean ± SD; Vehicle Only = 277.14 ± 113.65; 50 µg = 155.42 ± 37.54; 200 µg = 224.06 ± 89.21; 800 µg = 216.57 ± 100.55 mg/100 ml). (a priori t-test: Control vs. 50 µg, t = 3.23, df = 12; Control vs. 200 µg, t = 1.04, df 17; Control vs. 800 µg, t = 1.09, df = 16).

These findings indicate that intramuscular injections of IGF-I vector (SK-7331-IGF-I-SK2) reduce diabetic hyperglycemia and increase body and muscle weight suggesting that IGF-I expression levels are sufficient to trigger an anabolic effect. The finding that the vector injected, but not the contralateral, gastrocnemius significantly increases in weight suggests a difference in local IGF-I concentration in the two muscles.

### Effect of Substitution of the hGH 3' UTR for the skeletal actin 3' UTR in skeletal actin - IGF-I transgenes on circulating concenetrations of hIGF-I in transgenic mice

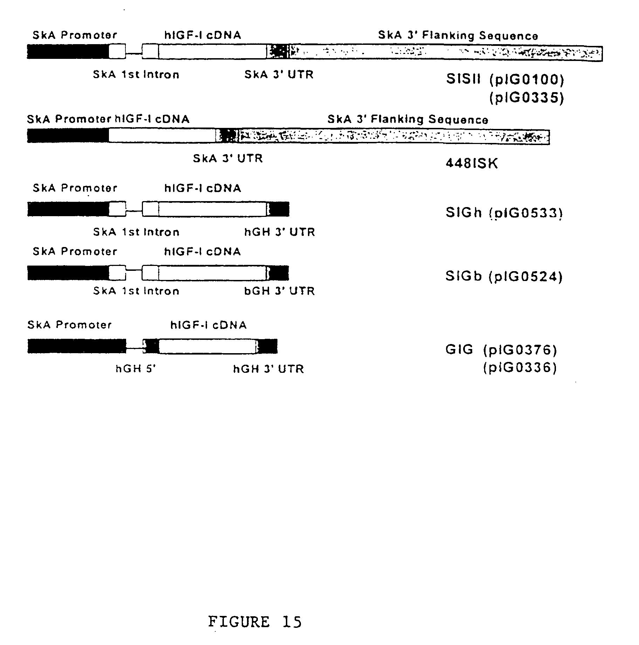

Transgenic mice containing the skeletal actin-IGF-I transgenes described in Figure 15 were generated. Serum samples were obtained and assayed for hIGF-I. Results (Table IV) clearly demonstrate that transgenes containing the hGH 3' UTR elicit increased concentraions of hIGF-I in circulation relative to transgenes containing the skeletal actin 3' UTR. Other variables such as the presence/abscence or origin of intron and the origin of the 5' UTR appear to have little or no effect.

**Table IV**

| Human IGF-I concentrations in serum of mice carrying skeletal actin - IGF-I transgenes. | | |
|---|---|---|
| Transgene | Animal ID | hIGF-I (ng/ml) |
| SISII | 2813 | 3.0 |
| 448ISK | 8219 | 6.5 |
| 448ISK | 8226 | ND¹ |
| 448ISK | 8230 | ND |
| SIGh | 2950 | 292.9 |
| SIGh | 5196 | 30.3 |
| GIG | 2338 | 253.7 |
| GIG | 2360 | 94.5 |
| Non-transgenic control | | ND |

| | | |
|---|---|---|
| ¹ND - Not detectable (assay sensitivity is approximately 1 ng/ml). | | |

As is shown by the data in the table, the GH 3' UTR sequences result in greatly enhanced serum concentrations (i.e., enhanced secretion) of the encoded polypeptide as compared to the use of 3' sequences, such as skeletal actin 3' UTR, which provide higher retention of the product in the tissue. Thus, selection of 3' UTR sequences having appropriate secretion or retention promoting properties provides the ability to control the localization of the encoded product.

### Enhanced Vector Expression in Intact Muscle

Intact plasmid DNA in a sterile 20% sucrose solution (wt/vol) can be injected into mature avian or mammalian muscle. Following a single injection the vector DNA is stable for at least 30 days as a non-integrated extrachromosomal circular DNA in muscle nuclei and, is transcriptionally active. Wolf et al., *Science,* vol. 247, pp. 1465-1468 (1990). However, greater than 99% of the injected DNA is degraded in muscle under the Wolff protocol (Wolff, et al, *BioTechniques* 11:4374-485 (1991)). This protocol can be improved by increasing the uptake of plasmid DNA into muscle and reducing vector degradation. The procedure of the present invention can use expression vector DNA coated with the relevant transcriptional regulatory factors, the human serum response factor and other human associated nuclear proteins, such as histone, and transcription initiation factors to enhance uptake and stability. The regulatory proteins protect the DNA against muscle nucleases and facilitate the uptake of the protein coated DNA into myogenic nuclei.

The expression vector forms a protein/DNA complex by the sequence specific binding of the serum response factor with the inner core CCXXXXXXGG (where X can be either A or T; SEQ ID NO. 6) of the serum response element and by the addition of histone. The interaction with the inner core of the promoter facilitates myogenic cell type restricted expression of the skeletal α-actin gene. The serum response factor, transcription initiation factor, transregulatory factor and histones are added to the expression vector by an in vitro binding reaction to form a reconstituted protein/DNA complex.

### Coating the Expression Vector System

A specific formulation involves coating the vector with elements of the transcription initiation complex and histone. This formulation is used both to enhance delivery of the vector to the cell and to enhance expression of the vector within the cell.

The following protocol was used to bacterially express and purify human serum response factor (SRF). Plasmid pARSRF-Nde is a T7 polymerase vector (Studier, F.W. and Moffatt, *J. Mol. Biol.* 189:113-130 (1986)) which produced full-length SRF protein upon IPTG (isopropyl-B-D-thiogalactopyranoside) induction. (Manak et al., *Genes and Development* 4:955-967 (1990)). E. coli BL21 harboring the plasmid was grown at 37°C to an OD₆₀₀ of 0.4 in TYP medium supplemented with ampicillin (50 µg/ml). Synthesis of SRF was then induced with 1mM IPTG for 2.0 hr, after which cells were spun down, washed once in TE buffer (10 mM Tris-HCl, 1mM EDTA, pH 7.0) and resuspended in a 40X packed cell volume and dialyzed against (10 mM HEPES [N-2 hydroxyethylpiperzine-N-2-ethansulfonic acid, pH 7.4], 60 mM KCl, 1mM 2-mercaptoethanol 0.5 mM EDTA, 0.5 mM phenylmethylsulfonyl fluoride and 10% glycerol). Cells were disrupted on ice by sonication. The lysate was clarified by centrifugation (15,000 xg for 20 min.) and the high speed supernatant containing overexpressed SRF was stored at -80C. Partial purification of SRF was done as follows. A 10 ml amount of the lysate was applied to a 10 ml phosphocellulose column equilibrated with column buffer (same as dialysis buffer as described above) and 0.05% Nonidet P-40. The flow through fractions were collected and applied to a 5-ml heparin agarose column. The column was washed with 0.35 M KCl and SRF was eluted with 0.5 M KCl. SRF was then dialyzed and stored at -80°C.

Approximately, a ratio by weight of 5 to 1 SRF protein to expression vector DNA was allowed to incubate together in a solution containing 10 mM. Tris-HCl (pH 8.0, 0.1 mM EDTA, 2mM dithiothreitol, 5% glycerol plus 100 mM KCl. The binding of SRF to the actin promoter has been verified by DNA binding assays and by nuclease footprint protection assays as shown in the art. Transcription initiation factors such as the TATA box protein (TBP) and other initiation factors such as TFIIB, E and F are eluted from purified HeLa cell nuclei by the protocol of Dignam et al., *Mol. Cell. Biol.* 10:582-598 (1983) with 0.42M KCl in the above dialysis buffer. Nuclear lysates containing transcription initiation factors are mixed together with the SRF-DNA plasmid at a ratio of 10 parts protein to one part SRF-DNA to help form a preinitiation complex which is dialyzed for 24 hours. Finally, a crude histone preparation which is stripped from HeLa nuclei in 6M urea, 2M NaCl is dialyzed against low salt dialysis buffer. The full complement of histone are slowly added to a final ratio of 1 to 1 (histone to the SRF-protein DNA complex) to form nucleosome particles over nonprotected DNA. The addition of histone will protect regions of DNA to a greater extent than naked DNA from cellular nucleases.

The nucleoprotein complex is then further formulated with a lipid base, nonaqueous base and/or liposomes for direct injection into muscle. Because of the abundance of specific transcription factors, which contain nuclear targeting sequences, expression vector DNA is readily delivered, and taken up into muscle nuclei.

The vector can also be prepared in a formulation with other DNA binding compounds. For example, the vector can be prepared with polyvinyl pyrrolidone (PVP). PVP is a synthetic polymer consisting of linear 1-vinyl-2-pyrrolidone groups. PVP is commercially available with various degrees of polymerization and molecular weights. Pharmaceutical grade PVP is marketed under the trade names Plasdone (International Specialty Products, ISP) and Kollidon (BASF). ISP describes the typical properties of Plasdone C-30 in its product literature. Plasdone C-30 has a weight average molecular weight of 50,000 g/mol.

PVP is found to interact with DNA by hydrogen bonding. PVP is also found to protect DNA *in vitro* from nuclease (DNase 1) degradation. Reporter genes (CMV-CAT or CMV-β-gal) were formulated in PVP solutions and injected into rat tibialis muscles after surgical exposure. The results showed that DNA formulated at 3 mg/mL in 5% PVP in 150 mM NaCl led to the highest enhancement of gene expression over DNA formulated in saline. The levels of gene expression using lower molecular weight PVP (Plasdone C-15) were approximately 2-fold lower than levels of gene expression using formulations made with Plasdone C-30. When rat tibialis muscles were injected with DNA formulated in either saline or 5% PVP (Plasdone C-30), immunochemical staining for β-galactosidase revealed that the staining was more widely distributed in muscles treated with the formulated DNA. The staining also showed that the PVP formulation resulted in an increase in the number of cells expressing β-gal and that these cells were distributed over a larger area as compared to DNA injected in saline. It is suggested that the increased tissue dispersion of DNA using PVP formulations is due to a hyper-osmotic effect in the muscle. DNA (3 mg/mL) in 5% PVP (Plasdone C-30) in 150 mM NaCl exerts an osmotic pressure of 341 ± 1 mOsm/kg H₂O.

An exemplary formulation of the hIGF-I plasmid is a three-vial system, with product components to be mixed just prior to use. The product components are:
1. Human IGF-I plasmid in sterile water;
2. Lyophilized PVP (polyvinylpyrrolidone; Plasdone C-30, Povidone U.S.P.); chemical formula (C₆H₉NO)ₙ;
3. 115 mM sodium citrate buffer (pH 4) in 5% NaCl.

The expression vector can also be delivered as described below.

### Administration

Administration as used herein refers to the route of introduction of a vector or carrier of DNA into the body. Administration can be directly to a target tissue or by targeted delivery to the target tissue after systemic administration. In particular, the present invention can be used for treating disease by administration of the vector to the body in order to establishing controlled expression of any specific nucleic acid sequence within tissues at certain levels that are useful for gene therapy.

The preferred means for administration of vector and use of formulations for delivery are described above. The preferred embodiment is by direct injection using needle injection or hypospray.

The route of administration of any selected vector construct will depend on the particular use for the expression vectors. In general, a specific formulation for each vector construct used will focus on vector uptake with regard to the particular targeted tissue, followed by demonstration of efficacy. Uptake studies will include uptake assays to evaluate cellular uptake of the vectors and expression of the tissue specific DNA of choice. Such assays will also determine the localization of the target DNA after uptake, and establishing the requirements for maintenance of steady-state concentrations of expressed protein. Efficacy and cytotoxicity can then be tested. Toxicity will not only include cell viability but also cell function.

Muscle cells have the unique ability to take up DNA from the extracellular space after simple injection of DNA particles as a solution, suspension, or colloid into the muscle. Expression of DNA by this method can be sustained for several months.

Delivery of formulated DNA vectors involves incorporating DNA into macromolecular complexes that undergo endocytosis by the target cell. Such complexes may include lipids, proteins, carbohydrates, synthetic organic compounds, or inorganic compounds. The characteristics of the complex formed with the vector (size, charge, surface characteristics, composition) determines the bioavailability of the vector within the body. Other elements of the formulation function as ligand which interact with specific receptors on the surface or interior of the cell. Other elements of the formulation function to enhance entry into the cell, release from the endosome, and entry into the nucleus.

Delivery can also be through use of DNA transporters. DNA transporters refers to molecules which bind to DNA vectors and are capable of being taken up by epidermal cells. DNA transporters contain a molecular complex capable of noncovalently binding to DNA and efficiently transporting the DNA through the cell membrane. It is preferable that the transporter also transport the DNA through the nuclear membrane. See, e.g., the following applications all of which (including drawings) are hereby incorporated by reference herein: (1) Woo et al., U.S. Serial No. 07/855,389, entitled "A DNA Transporter System and Method of Use,, filed March 20, 1992, now abandoned; (2) Woo et al., PCT/US93/02725, International Publ. WO93/18759, entitled "A DNA Transporter System and method of Use", (designating the U.S. and other countries) filed March 19, 1993; (3) a continuation-in-part application by Woo et al., entitled "Nucleic Acid Transporter Systems and Methods of Use", filed December 14, 1993, U.S. Serial No. 08/167,641; (4) Szoka et al., U.S. Serial No. 07/913,669, entitled "Self-Assembling Polynucleotide Delivery System", filed July 14, 1992 and (5) Szoka et al., PCT/US93/03406, International Publ. WO93/19768 entitled "Self-Assembling Polynucleotide Delivery System", (designating the U.S. and other countries) filed April 5, 1993.

Transfer of genes directly into muscle has been very effective. Experiments show that administration by direct injection of DNA into muscle cells results in expression of the gene in the area of injection. Injection of plasmids containing IGF-I results in expression of the gene for months at relatively constant levels. The injected DNA appears to persist in an unintegrated extrachromosomal state. This means of transfer is the preferred embodiment.

Another preferred method of delivery involves a DNA transporter system. The DNA transporter system consists of particles containing several elements that are independently and non-covalently bound to DNA. Each element consists of a ligand which recognizes specific receptors or other functional groups such as a protein complexed with a cationic group that binds to DNA. Examples of cations which may be used are spermine, spermine derivatives, histone, cationic peptides and/or polylysine. One element is capable of binding both to the DNA vector and to a cell surface receptor on the target cell. Examples of such elements are organic compounds which interact with the asialoglycoprotein receptor, the folate receptor, the mannose-6-phosphate receptor, or the carnitine receptor. A second element is capable of binding both to the DNA vector and to a receptor on the nuclear membrane. The nuclear ligand is capable of recognizing and transporting a transporter system through a nuclear membrane. An example of such ligand is the nuclear targeting sequence from SV40 large T antigen or histone. A third element is capable of binding to both the DNA vector and to elements which induce episomal lysis. Examples include inactivated virus particles such as adenovirus, peptides related to influenza virus hemagglutinin, or the GALA peptide described in the Skoka patent cited above.

Administration may also involve lipids. The lipids may form liposomes which are hollow spherical vesicles composed of lipids arranged in unilamellar, bilamellar, or multilamellar fashion and an internal aqueous space for entrapping water soluble compounds, such as DNA, ranging in size from 0.05 to several microns in diameter. Lipids may be useful without forming liposomes. Specific examples include the use of cationic lipids and complexes containing DOPE which interact with DNA and with the membrane of the target cell to facilitate entry of DNA into the cell.

Gene delivery can also be performed by transplanting genetically engineered cells. For example, immature muscle cells called myoblasts may be used to carry genes into the muscle fibers. Myoblasts genetically engineered to express recombinant human growth hormone can secrete the growth hormone into the animal's blood. Secretion of the incorporated gene can be sustained over periods up to 3 months.

Myoblasts eventually differentiate and fuse to existing muscle tissue. Because the cell is incorporated into an existing structure, it is not just tolerated but nurtured. Myoblasts can easily be obtained by taking muscle tissue from an individual who needs gene therapy and the genetically engineered cells can also be easily put back with out causing damage to the patient's muscle. Similarly, keratinocytes may be used to deliver genes to tissues. Large numbers of keratinocytes can be generated by cultivation of a small biopsy. The cultures can be prepared as stratified sheets and when grafted to humans, generate epidermis which continues to improve in histotypic quality over many years. The keratinocytes are genetically engineered while in culture by transfecting the keratinocytes with the appropriate vector. Although keratinocytes are separated from the circulation by the basement membrane dividing the epidermis from the dermis, human keratinocytes secrete into circulation the protein produced.

Delivery may also involve the use of viral vectors. For example, an adenoviral vector may be constructed by replacing the E1 region of the virus genome with the vector elements described in this invention including promoter, 5'UTR, 3'UTR and nucleic acid cassette and introducing this recombinant genome into 293 cells which will package this gene into an infectious virus particle. Virus from this cell may then be used to infect tissue *ex vivo* or *in vivo* to introduce the vector into tissues leading to expression of the gene in the nucleic acid cassette.

The chosen method of delivery should result in expression of the gene product encoded within the nucleic acid cassette at levels which exert an appropriate biological effect. The rate of expression will depend upon the disease, the pharmacokinetics of the vector and gene product, and the route of administration, but should be between 1-1000 mg/kg of body weight/day. This level is readily determinable by standard methods. It could be more or less depending on the optimal dosing. The duration of treatment will extend through the course of the disease symptoms, possibly continuously. The number of doses will depend upon disease delivery vehicle and efficacy data from clinical trials.

### Animal Safety/Toxicology Studies

### A. Acute 7-day and Subchronic 28-day Toxicity Studies

Acute 7-day and subchronic 28-day toxicity studies were conducted in dogs in compliance with the Good Laboratory Practice (GLP) Regulations of the United States Food and Drug Administration (21 CFR Part 58). The test articles and vehicle used in these studies were manufactured under cGMP procedures. Dogs were used because the mature human IGF-I (hIGF-I) which is expressed by the plasmid is identical to canine IGF-I.

The objective of the 7-day acute study was to investigate the potential acute toxicity of hIGF-I plasmid following a single intravenous injection in the dog. Four groups of beagle dogs, each consisting of two males and two females, were injected intravenously with the test article, hIGF-I plasmid formulated in polyvinylpyrrolidone (PVP), at dosage levels of 0.1, 1.0, and 12.0 mg/kg. The highest dose level was selected based on the maximum solubility of the test article in the vehicle and the total volume allowed for injection in dogs. The low dose corresponds to the minimum effective dose in preclinical animal studies in rodents.

A control group received the vehicle (PVP) only at the highest dose used with the test article. Two additional recovery groups (two males and two females in each) treated with the highest dose of test article and control animals were kept for another week. The dogs were sacrificed on day 8 after injection, and the recovery groups were sacrificed on day 15 after injection.

An intravenous route of administration was used to mimic a "worst-case" scenario of systemic exposure of the test article. Mortality checks were performed twice daily throughout the study, and detailed examinations for clinical signs were performed hourly for the first four hours after dosing and daily during the observation period. Body weights were measured twice weekly during the last week of acclimation and throughout the observation period. Laboratory investigations (hematology, clinical biochemistry and urinalysis) were performed during the pretreatment period and on samples collected on days 2, 7, and 14 for all surviving animals. A complete necropsy was conducted on all animals, and selected organs including muscles were weighed.

There were neither abnormal clinical signs nor effects on body weight, food consumption, hematology, clinical biochemistry or urinalysis parameters. In addition, there were no differences in organ weights or gross pathological findings related to hIGF-I plasmid. Clinical signs consistent with histamine release were observed in control and high dose animals. These signs lasted for approximately two hours and were consistent with previous reports of histamine release in response to PVP observed in dogs. Thus, the signs were attributed to the PVP present in the dose formulations and not to the hIGF-I plasmid.

The objective of the subchronic study was to investigate the potential toxicity of hIGF-I plasmid during weekly intramuscular injection to beagle dogs for four weeks, followed by a four week recovery period. The intramuscular route is the intended route of administration in humans. Dogs were injected intramuscularly once weekly for four weeks with 0.1, 1.0, and 6.0 mg/kg. Each group consisted of three dogs per sex. Additional recovery groups (two dogs/sex) at the highest dose and control animals were observed for an additional 28 days. Mortality checks were performed at least twice daily throughout the study, and examinations for clinical signs of ill-health or reaction to treatment were performed at least twice daily following initiation of treatment. Individual body weights were determined on the day of randomization and weekly during the treatment and recovery periods. Food consumption was measured daily during the treatment and recovery periods. Ophthalmoscopy was performed once prior to the start of treatment and again during the last week of treatment (Week 4) and the last week of the recovery period (Week 8). Cardiovascular studies (electrocardiograms and systolic blood pressure measurements) and laboratory investigations (hematology, clinical biochemistry and urinalysis) were performed once prior to the start of treatment and again during weeks 4 and 8. In addition, serum samples were obtained on the same occasions and stored for possible future analysis. A complete necropsy was conducted on all animals sacrificed at the end of the treatment period. Selected organs were weighed, and a complete list of tissues was retained and microscopically evaluated.

There were neither abnormal clinical signs nor effects on body weight, food consumption, blood pressure, electrocardiograms, hematology, clinical biochemistry, urinalysis parameters, or ocular changes which were considered related to hIGF-I plasmid. As in the acute study, clinical signs consistent with histamine release were observed in control and high dose animals following administration of the control or test article. In response to occasional (4 out of 16) severe reactions, epinephrine was administered intravenously to prevent mortality. As before, these reactions were attributed to the PVP present in the test article and not to hIGF-I plasmid.

A pilot exploratory study conducted in two dogs confirmed that the observed clinical signs were due to histamine release. The dogs were injected intramuscularly with the vehicle at the high dose level (6 mg/kg) that elicited the clinical signs of histamine release. One of the dogs was pretreated with an H₁ histamine receptor blocker, diphenhydramine hydrochloride (Benadryl^{R}, 1 mg/kg). Both dogs developed the clinical signs, and pretreatment with the histamine antagonist did not abrogate the signs. Blood samples were analyzed for histamine levels, and they were approximately 100-fold or more higher than pretreatment levels in both dogs. These results suggest that the dosage of histamine blocker was inadequate. We believe that the effects seen in dogs are species specific and are unique to dogs. Experience with PVP used as a blood expander has not shown similar clinical signs in humans. There were no differences in organ weights or gross or histopathological findings which were considered to be related to hIGF-I plasmid.

Thus, administration of hIGF-I plasmid by weekly intramuscular injection for four weeks produced no evidence of toxicity at doses up to 6 mg/kg/occasion. Clinical signs consistent with histamine release observed in control and high dose animals were attributed to the polyvinylpyrrolidone present in the dose formulations and not to hIGF-I plasmid.

### B. Assay of Canine Serum for Anti-hIGF-I and Anti-DNA Antibodies

Serum samples obtained from dogs treated with hIGF-I plasmid in the subchronic (28 day) toxicity study were assayed for the presence of antibodies to rhIGF-I and double stranded (ds) DNA. Dogs were injected intramuscular with hIGF-I plasmid at dosages of 0 (vehicle control), 0.1, 1.0 and 12.0 mg/kg every 7 days for a period of 28 days. Serum samples (see outline in Table V) were obtained prior to the initiation of dosing (pre-bleed), at the end of dosing (day 27), and after a 28 day recovery phase (day 55). A total of 76 samples were assayed.

**Table V**

| Outlines of serum samples assayed for antibodies to hIGF-I and to DNA | | | | | |
|---|---|---|---|---|---|
| Dosage | Pre-bleed | Day 27 | Day 55 | | |
| | **F** | **F** | **M** | **F** | |
| **sex** | **M** | | | | **M** |
| Vehicle | 5^{a} | 5 | 5 | 3 | |
| control | 5 | | | | 3 |
| 0.1 mg/kg BW | 3 | 3 | 3 | | |
| | 3 | | | | |
| 1.0 mg/kg BW | 3 | 3 | 3 | | |
| | 3 | | | | |
| 12.0 mg/kg BW | 5 | 5 | 5 | 3 | |
| | 5 | | | | 3 |

| | | | | | |
|---|---|---|---|---|---|
| ^{a}Numbers represent the number of serum samples that were collected and assayed. | | | | | |

Antibodies to rhIGF-I were assayed using standard ELISA procedures. Results indicated that serum samples from treated dogs contained no detectable antibodies to rhIGF-I. Antibodies to dsDNA were assayed using an ELISA kit (The Binding Site, Inc., Birmingham, U.K.) designed to quantitate antibodies to dsDNA in human serum and was modified to quantify antibodies in dog serum. The anti-human IgG HRP conjugate was replaced with rabbit anti-dog IgG HRP conjugate as the second antibody. Results indicated that no serum samples from treated dogs contained detectable antibodies to ds DNA.

### Pharmacokinetics/Biodistribution Studies

### A. Time-Course of Expression of rhIGF-I in Rat Skeletal Muscle

Expression of rhIGF-I in tibialis anterior muscles of rats was determined at time points following intramuscular injection of hIGF-I plasmid. Human IGF-I plasmid formulated in polyvinyl pyrrolidone was injected bilaterally into tibialis anterior muscles (150 µg DNA/muscle) of male Fisher 344 rats (approximately 125 g BW). Rats were randomly divided into two groups with animals in one group receiving every other day injections of the immunosuppressant cyclosporine A (5 mg/kg BW) into the gluteus muscle for the duration of the experiment. Rats from each group were sacrificed at 24 hours, 48 hours, 7 days, 14 days, and 28 days following injection of hIGF-I plasmid (n=5-6 rats/group/time point). Tibialis anterior muscles were harvested at these times and analyzed for expression of rhIGF-I by immunoradiometric assay (Diagnostic Systems Laboratories, Inc., Webster, TX).

Reverse transcriptase PCR analysis of total RNA from injected muscles did not reveal expression of hIGF-I mRNA at 24 or 48 hours post-injection. Expression of hIGF-I mRNA was observed at days 7, 14, and 28. Results for intramuscular content of rhIGF-I at 7, 14, and 28 days post-injection show that intramuscular content of rhIGF-I was similar at approximately 1.5-2.5 ng/g muscle between days 7 and 14 post-injection and decreased to approximately 35 percent of day 7 values by 28 days post-injection. Treatment with cyclosporine A to suppress the immune response did not affect (p>.10) intramuscular rhIGF-I content nor were antibodies to hIGF-I detected in serum of rats not receiving cyclosporine A treatment at 28 days post-injection. Together, these results suggest that the decrease in intramuscular rhIGF-I was not due to a host immune response. Human IGF-I was not detected in serum samples from injected rats at any time point.

### B. Determination of the Pharmacokinetics and Tissue Distribution of hIGF-I Plasmid

The objective of these studies was to determine the pharmacokinetics and tissue distribution of hIGF-I plasmid following administration. Like the canine IGF-I, the mature guinea pig IGF-I polypeptide is identical to human IGF-I, making the guinea pig a suitable species to study the pharmacokinetics and biodistribution of hIGF-I plasmid. Two groups of Hartley guinea pigs were each injected once with either a low dose (0.1 mg/kg) or a high dose (1.5 mg/kg) of hIGF-I plasmid formulated in 5% PVP by intramuscular or intravenous injections (50 males and 50 females per route of administration).

Five animals per sex from each treatment group were sacrificed at the following time intervals: 30 minutes, 1 hour, 6 hours, 12 hours, 1 day, 2 days, 1 week, 4 weeks and 3 months. As a control, one group of 10 male and 10 female guinea pigs per route of administration received the vehicle at the same volume/weight ratio as the high dose-treated group.

Gonads, lymph nodes, liver, spleen, kidney, lungs, heart, brain, bone marrow, muscle, and blood were collected at each sacrifice point. The blood was stored at 5°C, and the tissues were frozen in liquid nitrogen and stored at -70°C. DNA from blood samples was analyzed for the presence of the human IGF-I plasmid using a sensitive polymerase chain reaction (PCR) assay. If the plasmid was detected in the blood, the selected tissues were presumed to be positive and were not analyzed. If the plasmid was not detected in the blood sample, the DNA from the tissue samples was amplified in duplicate. One sample of each duplicate was spiked with the test plasmid at a copy number near the limit of detection to demonstrate the absence of any polymerase chain reaction (PCR) inhibition. Samples from animals treated using the high dose intramuscular and the intravenous routes have not yet been analyzed. Any positive tissue can be analyzed for human IGF-I messenger RNA to determine gene expression.

PCR evaluation of the blood samples from animals sacrificed through the second day following test article dosing indicated the presence of the DNA plasmid. By day 7 after dosing, the plasmid had disappeared from the blood. PCR analysis of the samples (gonads, kidneys, liver, heart, and muscle tissues) from animals from the animals sacrificed three months after intramuscular injection of a low dose of hIGF-I plasmid (0.1 mg/kg) indicated elimination of most of the test plasmid at the injection site and in the peripheral systemic locations.

No clinical signs of toxicity were observed in any animal of either sex during the course of the study. All animals survived to the scheduled sacrifices. The animals for which terminal body weights were recorded (animals sacrificed after day 1) gained weight from the time of dosing to the time of sacrifice. There were no apparent significant chemically-related effects on body weight. Gross examination of selected tissues at necropsy revealed no abnormal findings at any time point with one exception. Brown foci on all lobes of the lungs were observed in one low dose female; however, this finding was not thought to be related to treatment with the test article.

In general, the data indicated that the hIGF-I plasmid is eliminated after three months. Out of a total of fifty tissues analyzed from ten animals given intramuscular injections of the low dose plasmid, only three positive signals were noted: one ovary, one liver and one muscle. The signals were sporadic and did not appear to be tissue-specific. All negative control tissues gave negative results.

Therefore, based on the complete lack of test article-related mortality, clinical signs of toxicity, effects of body weight, or gross lesions at necropsy three months following exposure, the test article (as administered) is not toxic at the doses tested.

### Cell Transfection and Transformation

One aspect of the present invention includes cells transfected with the vectors described above. Once the cells are transfected, the transformed cells will express the protein or RNA encoded for by the nucleic acid cassette. Examples of proteins include, but are not limited to polypeptide, glycoprotein, lipoprotein, phosphoprotein, or nucleoprotein.

The nucleic acid cassette which contains the genetic material of interest is positionally and sequentially oriented within the vectors such that the nucleic acid in the cassette can be transcribed into RNA and, when necessary, be translated into proteins or polypeptides in the transformed cells.

A variety of proteins can be expressed by the sequence in the nucleic acid cassette in the transformed cells. Those proteins which can be expressed may be located in the cytoplasm, nucleus, membranes (including the plasmalemma, nuclear membrane, endoplasmic reticulum or other internal membrane compartments), in organelles (including the mitochondria, peroxisome, lysosome, endosome or other organelles), or secreted. Those proteins may function as intracellular or extracellular structural elements, ligand, hormones, neurotransmitter, growth regulating factors, differentiation factors, gene-expression regulating factors, DNA-associated proteins, enzymes, serum proteins, receptors, carriers for small molecular weight organic or inorganic compounds, drugs, immunomodulators, oncogenes, tumor suppressor, toxins, tumor antigens, or antigens. These proteins may have a natural sequence or a mutated sequence to enhance, inhibit, regulate, or eliminate their biological activity. A specific example of a protein to be expressed is hIGF-I.

In addition, the nucleic acid cassette can code for RNA. The RNA may function as a template for translation, as an antisense inhibitor of gene expression, as a triple-strand forming inhibitor of gene expression, as an enzyme (ribozyme) or as a ligand recognizing specific structural determinants on cellular structures for the purpose of modifying their activity. Specific examples include RNA molecules to inhibit the expression or function of prostaglandin synthase, lipooxenganse, histocompatibilty antigens (class I or class II), cell adhesion molecules, nitrous oxide synthase, β₂ microglobulin, oncogenes, and growth factors.

The compounds which can be incorporated are only limited by the availability of the nucleic acid sequence for the protein or polypeptide to be incorporated. One skilled in the art will readily recognize that as more proteins and polypeptides become identified they can be integrated into the vector system of the present invention and expressed in animal or human tissue.

Transfection can be done either by *in vivo* or *ex vivo* techniques. For example, muscle cells can be propagated in culture, transfected with the transforming gene, and then transplanted into muscle tissue. Alternatively, the vectors can be administered to the cells by the methods discussed above.

### Methods of Use

### A. Treatment with Growth Hormone

Growth hormone is normally produced and secreted from the anterior pituitary and promotes linear growth in prepuberty children. Growth hormone acts on the liver and other tissues to stimulate the production of insulin-like growth factor I. This factor is, in turn, responsible for the growth promoting effects of growth hormone. Further, this factor serves as an indicator of overall growth hormone secretion. Serum IGF-I concentration increases in response to endogenous and exogenous administered growth hormone. These concentrations are low in growth hormone deficiency.

Insulin-like growth factors are one of the key factors that potentiate muscle development and muscle growth. Myoblasts naturally secrete IGF-I/IGF-II as well as its cognate binding proteins during the onset of fusion. This process coincides with the appearance of muscle specific gene products. In terminally differentiated muscle, signals propagated from passive stretch induced hypertrophy induce the expression of IGF genes. Many of the actions of IGFs on muscle result from interactions with the IGF-I receptor.

The intramuscular injection of an expression vector containing the sequence for IGF-I (for example, pIG0552) can be used to treat growth disorders. Vectors are designed to control the expression of IGF-I in a range of 100-400 ng/ml. Since intramuscular expression of vectors leads to expression of the product encoded by the nucleic acid cassette for several months, this method provides a long-term inexpensive way to increase systemic blood concentration of IGF-I in patients with growth hormone deficiency.

### B. Effect of IGF-I Vector Expression on Disuse Atrophy

Hindlimb suspension is a common experimental procedure used to induce atrophy of the calf muscles. The effects of hindlimb suspension are similar to those induced by cast immobilization and prolonged exposure to zero gravity.

Mice (12/group) were injected into the gastrocnemius and tibialis anterior muscles with either IGF-I containing vector (IGF-I) or control plasmid (PLAS) at days 0 and 7 of the suspension phase. The vectors were formulated at 3 mg/ml in poly-vinyl pyrrolidone (PVP) solution and administered at doses of 25 µl (75 µg DNA) into the tibialis anterior muscle and 50 µl (150 µg DNA) into the gastrocnemius muscle. This corresponds to a dosage of approximately 1 µg DNA/mg wet muscle weight.

Contractile force (strength) measurements and muscle weights were taken 1-2 days after cessation of hindlimb suspension. Animal not subjected to hindlimb suspension (NORM) were included for comparison.

Results, shown in Table VI, indicate that hindlimb suspension elicited an approximately 20-25% loss of muscle mass and strength and that treatment with IGF-I vector formulation reduced these effects (p<.10).

**Table VI**

| Mean Values for Selected Parameters | | | | | | | |
|---|---|---|---|---|---|---|---|
| Treatment | BW(g) | Tibialis weight (mg) | Tibialis (% body weight) | Tw tension (g) | Tet tension (g) | Gastroc. weight (mg) | Gastroc. (% body weight) |
| PLAS | 27.33 | 47.39 | .174 | 21.55 | 69.14 | 123.69 | .454 |
| IGF-I | 27.09 | 50.79 | .187 | 24.00 | 77.88 | 128.96 | .476 |
| NORM | 31.14 | 59.72 | .193 | 22.19 | 81.00 | 163.01 | .525 |

### C. Effects of IGF-I Vector Expression Following Crush Denervation

Sciatic nerve crush is a commonly used and well characterized model for elucidating the processes involved in degeneration and regeneration of neuromuscular function following trauma (M. Jaweed, 1994, The Physiological Basis of Rehabilitation, Downey et al., eds., p.543-561. Crush injury to the sciatic nerve results in rapid degeneration of axons distal to the lesion, loss of nerve conduction, and atrophy in the denervated muscle(s).

Early events in nerve regeneration begin within hours after crush injury initiating an ordered series of regenerative processes leading to re-establishment of neuromuscular synapses after 14-21 days and resumption of normal neuromuscular transmission after approximately 6 weeks in rodents. As a result of denervation, approximately 40-50% atrophy of affected myofibers and a concomitant decrease in isometric contractile force are observed after 14 days with eventual recovery to 80-90% of normal. Recoupment of muscular mass to pre-injury states requires several months.

Previous studies in rodents have indicated that daily administration of rhIGF-I protein can enhance recovery of neuromuscular function following sciatic nerve crush.

Mature ICR strain mice were subjected to either unilateral sham (SHAM) operation or sciatic nerve crush. IGF-I containing vector formulation (IGF-I) or control plasmid (PLAS) was injected into the tibialis anterior and gastrocnemius muscles of the operated limb. Mice in the respective groups were subsequently injected with either IGF-I formulation or control plasmid formulation every 7 days thereafter.

The vectors were formulated at 3 mg/ml in polyvinyl pyrrolidone (PVP) solution and administered at doses of 25 µl (75 µg DNA) into the tibialis anterior muscle and 50 µl (150 µg DNA) into the gastrocnemius muscle. This corresponds to a dosage of approximately 1 µg DNA/mg wet muscle weight.

Analyses for contractile force, muscle weight, electromyographic (EMG) activity, and nerve conduction velocity (NCV) were conducted at 14 day intervals following nerve crush. Measurement of EMG activity and NCV were performed using a Dantec Neuromatic 2000 EMG/EP system.

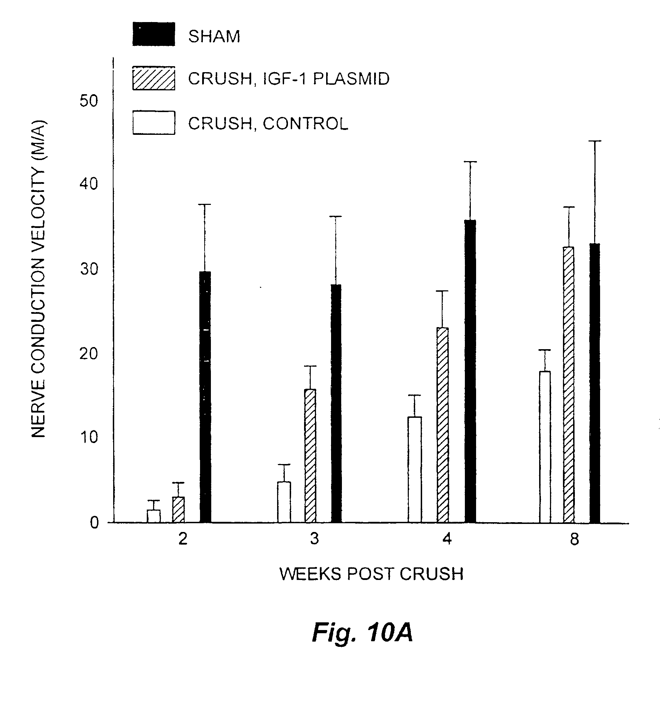

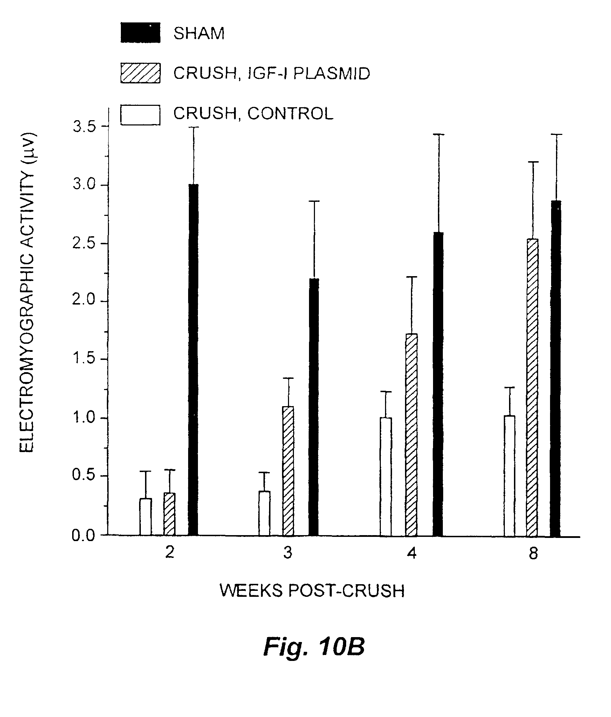

Sciatic nerve crush elicited marked muscle atrophy along with loss of nerve conduction and EMG activity (Figures 10A & B). No significant differences in these parameters were noted between hIGF-I plasmid-treated and control animals at two weeks post-crush. However, treatment with hIGF-I plasmid elicited a modest improvement in gastrocnemius mass at three weeks post-crush along with striking improvements in EMG activity and NCV beginning three weeks post-crush. These data suggest that the beneficial effects of hIGF-I plasmid are manifested relatively early (i.e., prior to three weeks) in the regenerative process.

These results indicate that expression from the IGF-I containing vector formulation enhances recovery from sciatic nerve crush.

### D. Treatment of Muscle Atrophy Due To Age

Growth hormone levels decline with increasing age. The levels in healthy men and women above age of 55 are approximately one third lower than the levels in men and women 18 to 33. This is associated with a decrease in the concentration of IGF-I. The decline in growth hormone and IGF-I production correlate with the decrease in muscle mass, termed senile muscle atrophy, and increase in adiposity that occur in healthy human subjects. Administering growth hormone three times a week to healthy 61 to 81 year old men who had serum levels below those of healthy younger men increased the serum IGF-I levels to within the range found in young healthy adults. This increased level led to increased muscle mass and strength and reduced body fat. The secretion of growth hormone is regulated by a stimulatory (growth hormone releasing hormone) and an inhibitory (somatostatin) hypothalamic hormone.

The convenient cloning sites in the expression vectors of the present invention are used to construct vectors containing human growth hormone CDNA sequence, the human growth hormone releasing hormone (GHRH), or IGF-I. This versatility is important since the GHRH, GH, and IGF-I, while having equivalent desired effects on muscle mass, may have different side effects or kinetics which will affect their efficacy. The expression of the growth factor releasing hormone might be more advantageous than the expression of either IGF-I or the growth hormone vectors transcripts. Since GHRH is reduced in the elderly it appears to be responsible for the lack of GH secretion rather than the anterior pituitary capability of synthesizing growth hormone, thus the increased expression of GHRH from muscle would increase GHRH levels in the systemic blood system and can allow for the natural diurnal secretion pattern of GH from the anterior pituitary. In this way, GHRH could act as the natural secretogogue, allowing for elevated secretion or release of GH from the hypothalamus of the elderly.

Thus, the application of vector systems described herein to express insulin-like growth factors through the injection of the pIG0552 or related vectors, the SK 733 IGF-I Sk2 vector, vectors expressing HG, or GHRH into adult muscle of the elderly is a long-term inexpensive way to increase systemic blood concentration of IGF-I in the elderly.

Administration of the vectors can be intravenously, through direct injection into the muscle or by any one of the methods described above. Dosages will depend on the severity of the disease and the amount of dosage is readily determinable by standard methods. The duration of treatment will extend through the course of the disease symptoms which can be continuously.

### E. Treatment of Human Muscle Atrophies Induced by Neurological Dysfunction

Insulin-like growth factors are also known neurotrophic agents which maintain neuronal muscular synapses, neuron integrity, and neuronal cell life under neurodegenerative conditions, and positively affect nerve regeneration. Since the expression vector driven genes are relatively insensitive to the innervation state of the muscle, they provide a direct and rather broad application for remedying certain kinds of human muscle atrophies caused by spinal cord injuries and neuromuscular diseases caused by drugs, diabetes, Type I disease, Type II diabetes, genetic diseases such as CHACOT-marie-tooth disease or certain other diseases. Moreover, IGF-I secretion can induce neurite outgrowth. In this treatment, the product of the vector acts as a neurotrophic agent secreted from injected muscle and as a hypertrophic agent to maintain muscle integrity.

Administration of the vectors can be intravenously, through direct injection or by any one of the methods described above. Dosages will depend on the severity of the disease and the amount of dosage is readily determinable by standard methods. The duration of treatment will extend through the course of the disease symptoms which can be continuously.

### F. Treatment of Diabetes

Insulin plays a central role in the regulation of carbohydrate, fat and protein metabolism. With diabetics, treatment with insulin can result in insulin resistance in which insulin treatment will not result in adequate metabolic control. This resistance can occur in the presence of circulating insulin or insulin-receptor antibodies or insulin-receptor abnormalities or episodically in patients with previously typical insulin-dependent diabetes mellitus. Therapeutic options are limited with patients suffering from severe insulin resistance.

IGF-I can be used in the treatment of insulin resistance. Treatment with IGF-I using the vectors of the present invention will achieve glycemic control by reversing hyperglycemia and ketoacidosis. Treatment with IGF-I will also improve the degree of insulin sensitivity. The convenient cloning sites in the expression vectors of the present invention are used to construct vectors containing the IGF-I cDNA sequence. Expression of IGF-I provides insulin-like metabolic effects. IGF-I shares sequence homology and biological properties with insulin.

Administration of the vectors can be intravenously, through direct injection or by any one of the methods described above. Dosages will depend on the severity of the disease and the amount of dosage is readily determinable by standard methods. The duration of treatment will extend through the course of the disease symptoms which can be continuously.

### G. Treatment of Peripheral Neuropathies

Peripheral neuropathies are degenerative processes of sensory and motor nerves that often result from diabetes, Type I diabetes, Type II diabetes, genetic disease such as CHACOT-marie-tooth disease, AIDS, inflammation and side-effects from anti-cancer and anti-viral drugs. Current treatment is limited to pain management, with no treatment directed at the underlying cellular causes. Use of recombinant IGF-I has been suggested to restore some of the degenerative processes in peripheral neuropathies and to alleviate some of the associated dysfunction.

Administration of a vector encoding IGF-I to localized muscles afflicted with peripheral neuropathy by direct injection or hypospray will aid in the regeneration of neurons, decrease pain sensations, and increase mobility of the affected site. The distinct advantage of vector administration of IGF-I is increased levels at needed sites with reduced numbers of administrations. In the diabetic patient dosage schedules may allow for the combined effect of alleviation of symptoms of peripheral neuropathy and increased efficiency of insulin.

Administration of the vectors can be intravenously, through direct injection or by any one of the methods described above. Dosages will depend on the severity of the disease and the amount of dosage is readily determinable by standard methods. The duration of treatment will extend through the course of the disease symptoms which can be continuously.

### H. Treatment of Osteoporosis

Osteoporosis is a common accelerated loss of bone mass that often accompanies aging. The decreased bone density associated with osteoporosis leads to an increased susceptibility to bone fractures. Treatment with IGF-I is associated with increased bone density. Administration of a vector encoding IGF-I to muscles by direct injection or hypospray will aid in the redeposition of bone and thereby decrease the risk of fractures.

Administration of the vectors can be intravenously, through direct injection or by any one of the methods described above. Dosages will depend on the severity of the disease and the amount of dosage is readily determinable by standard methods. The duration of treatment will extend through the course of the disease symptoms which can be continuously.

### Transgenic Swine

An additional embodiment of the present invention is the 5 generation of improved domestic livestock. Specifically, introduction of the vector pIG0552, or a vector expressing IGF-I of porcine, bovine or ovine derivation into oocytes of domestic swine by the method described above for the generation of transgenic mice will generate swine expressing IGF-I in myogenic tissue. These transgenic swine have the desired livestock characteristics of increased muscle mass and reduced fat.

In addition, by providing contiguous 3' NCR, IGF-I is buffered against outside genomic sequences and is thus more protected from position effects, when integrated into the genome. In addition, by providing natural terminating sequences, the additional regulatory sequences that mark the transcriptional domain of skeletal α-actin prevent read through transcription, improve tissue specificity, developmental timing and transcriptional activity. Presence of 3'NCR sequence allows for a single copy of the integrated vector to produce 40-50% of the transcriptional activity of the endogenous sequences.

### Improvement of Livestock

An additional embodiment of the present invention is the improvement of livestock by injection of IGF-I vector constructs, or similar constructs encoding other growth hormones, such as growth hormone or growth hormone releasing hormone. Muscle injection of vectors encoding IGF-I by hypodermic or hypospray administration will promote increased muscle mass and reduced body fat in important livestock species such as cattle, sheep, swine, rabbits, deer, fish and birds such as turkeys, chickens, ducks, and geese. Administration of the vectors cal also be through any one of the methods described above.

One skilled in the art will readily appreciate that the present invention is well adapted to carry out the objects and obtain the ends and advantages mentioned as well as those inherent therein. The vector systems along with the methods, procedures treatments and vaccinations described herein are presently representative of preferred embodiments are exemplary and not intended as limitations on the scope of the invention.

## Claims

1. A vector for expression of a nucleic acid sequence in a cell, comprising:
a nucleic acid cassette containing a nucleotide sequence encoding IGF-I;
a 5' flanking region including one or more sequences necessary for expression of said nucleic acid cassette, wherein said sequences include a promoter from a skeletal α-actin gene;
a linker connecting said 5' flanking region to a nucleic acid, said linker having a position for inserting said nucleic acid cassette, wherein said linker is not naturally associated with the 5' and 3' regions; and
a 3' flanking region, including a 3'UTR and optionally a 3'NCR, wherein said 3' flanking region is 3' to said position for inserting said nucleic acid cassette, and wherein said 3'UTR comprises a functional sequence from a growth hormone 3'-UTR.

2. The vector of claim 1, wherein said IGF-I is human IGF-I.

3. The vector of claim 2, wherein said nucleotide sequence encoding for human IGF-I is a synthetic sequence.

4. The vector of claim 3, wherein said nucleotide sequence encoding for human IGF-I has the sequence of SEQ ID NO. 4.

5. The vector of claim 1, wherein said promoter from a skeletal α-actin gene is from a chicken.

6. The vector of claim 1, wherein said promoter from a skeletal α-actin gene is from a human.

7. The vector of claim 1, wherein said growth hormone. 3'UTR is from a human growth hormone gene.

8. The vector of claim 1, wherein an ALU repeat or ALU repeat-like sequence is deleted from said 3' UTR.

9. The vector of claim 1, wherein said IGF-I is human IGF-I, said promoter from a skeletal α-actin gene is from a chicken, and said growth hormone 3'-UTR is from a human growth hormone gene.

10. The vector of claim 1, wherein said 5' flanking region or said 3' flanking region or both regulates expression of said nucleic acid cassette predominantly in a specific tissue.

11. The vector of claim 2, wherein said specific tissue is myogenic.

12. The vector of claim 1, wherein said 5' flanking region includes a promoter, a TATA box, a Cap site and a first intron and intron/exon boundary in appropriate relationship for expression of said nucleic acid cassette.

13. The vector of claim 12, wherein said 5' flanking region further comprises a 5' mRNA leader sequence inserted between said promoter and said nucleic acid cassette.

14. The vector of claim 1, wherein said vector further comprises an intron/5' UTR from a chicken skeletal α-actin gene.

15. The vector of claim 1, wherein said vector further comprises an antibiotic resistance gene. '

16. The vector of claim 1, wherein said vector comprises a nucleotide sequence which is the same as the nucleotide sequence of plasmid pIG0552 as shown in Table 1.

17. A formulation for delivery and expression of a human IGF-I gene in a cell, said formulation comprising
a vector of claim 1 in a solution having between 0.5% and 50% PVP.

18. The formulation of claim 17, wherein said solution includes about 5% PVP.

19. A non-human transgenic animal having a plurality of cells containing the vector of claim 1.

20. The transgenic animal of claim 19, wherein said cell is a germ or somatic cell.

21. A cell transformed with a vector of claim 1.

22. The transformed cell of claim 21, wherein said cell is myogenic.

23. A method for transfection of-a cell ex vivo, comprising the step of contacting said cell with a vector of claim 1 for sufficient time to transfect said cell.

24. The method of claim 23, wherein said contacting is performed in the presence of an about 5% PVP solution.

25. The method of claim 23, further comprising the steps of cotransfecting said vector with a selectable marker and selecting the transformed cells.

26. A method for *ex vivo* delivery and expression of a human IGF-I gene in a plurality of cells, comprising the steps of
(a) transfecting said plurality of cells *ex vivo* with a vector of claim 1, and
(b) incubating said plurality of cells under conditions allowing expression of a nucleic acid sequence in said vector, wherein said nucleic acid sequence encodes IGF-I.

27. The method of claim 26, wherein said IGF-I is hIGF-I and said cells are human cells.

28. The method of claim 27, wherein said contacting is performed in the presence of an about 5% PVP solution.

29. The use of a vector of claim 1, which is suitable for transfecting a cell in situ, as a medicament.

30. The use of a vector according to claim 1 in the manufacture of a medicament for the treatment of a disease or condition selected from the group consisting of muscle atrophy, osteoporosis, diabetes, neuropathy and growth disorders.

31. The use of claim 30, wherein said disease or condition is muscle atrophy secondary to lower motor neuron injury or disuse.

## Patentansprüche

1. Vektor zum Exprimieren einer Nucleinsäuresequenz in einer Zelle, zu dem gehören:
eine Nucleinsäurekassette, die für eine für IGF-I kodierende Nukleotidsequenz enthält;
eine 5'-Flankierungsregion, die ein oder mehrere Folgen aufweist, die zum Exprimieren der Nucleinsäurekassette benötigt werden, wobei die Folgen einen von einem skeletalen α-Actin-Gen stammenden Promotor enthalten;
ein Linker, der die 5'-Flankierungsregion an eine Nucleinsäure bindet, wobei der Linker eine Position zum Einfügen der Nucleinsäurekassette aufweist und der Linker nicht auf natürlichem Wege mit den 5'- und 3'-Regionen assoziiert ist; und
eine 3'-Flankierungsregion, mit einer 3'UTR und optional einer 3'NCR, wobei die 3'-Flankierungsregion eine Region 3' bis zu der Position zum Einfügen der Nucleinsäurekassette ist, und die 3'UTR eine funktionelle Sequenz aus einem Wachstumshormon 3'-UTR aufweist.

2. Vektor nach Anspruch 1, bei dem das IGF-I menschliches IGF-I ist.

3. Vektor nach Anspruch 2, bei dem die für menschliches IGF-I kodierende Nukleotidsequenz eine synthetische Sequenz ist.

4. Vektor nach Anspruch 3, bei dem die für menschliches IGF-I kodierende Nukleotidsequenz die Sequenz von SEQ ID NO. 4 aufweist.

5. Vektor nach Anspruch 1, bei dem der von einem skeletalen α-Actin-Gen stammende Promotor von einem Küken stammt.

6. Vektor nach Anspruch 1, bei dem der von einem skeletalen α-Actin-Gen stammende Promotor vom Menschen stammt.

7. Vektor nach Anspruch 1, bei dem das Wachstumshormon 3'UTR von einem menschlichen Wachstumshormon-Gen stammt.

8. Vektor nach Anspruch 1, bei dem aus der 3' UTR ein ALU-Repeat oder eine einem ALU-Repeat ähnelnde Sequenz entfernt ist.

9. Vektor nach Anspruch 1, bei dem der IGF-I menschlicher IGF-I ist, der von einem skeletalen α-Actin-Gen stammende Promotor von einem Küken stammt und das Wachstumshormon 3'-UTR von einem menschlichen Wachstumshormon-Gen stammt.

10. Vektor nach Anspruch 1, bei dem die 5'-Flankierungsregion oder die 3'-Flankierungsregion oder beide überwiegend ein Exprimieren der Nucleinsäurekassette in einem spezifische Gewebe regulieren.

11. Vektor nach Anspruch 2, bei dem das spezifische Gewebe myogen ist.

12. Vektor nach Anspruch 1, bei dem die 5'-Flankierungsregion einen Promotor, eine TATA-Box, eine Cap-Stelle und eine erste Intron- und Intron/Exon-Grenze enthält, die sich in einer für ein Exprimieren der Nucleinsäurekassette geeigneten Relation befinden.

13. Vektor nach Anspruch 12, bei dem die 5'-Flankierungsregion ferner eine 5'-mRNA Leadersequenz aufweist, die zwischen dem Promotor und der Nucleinsäurekassette eingefügt ist.

14. Vektor nach Anspruch 1, bei dem der Vektor ferner eine Intron/5'-UTR von einem skeletale Küken-α-Actin-Gen aufweist.

15. Vektor nach Anspruch 1, bei dem der Vektor ferner ein Antibiotika-Resistenzgen aufweist.

16. Vektor nach Anspruch 1, bei dem der Vektor eine Nukleotidsequenz aufweist, die mit der Nukleotidsequenz des in Tabelle 1 gezeigten Plasmids pIG0552 übereinstimmt.

17. Formulierung zum Liefern und Exprimieren eines menschlichen IGP-I-Gens in einer Zelle, wobei die Formulierung enthält:
einen Vektor nach Anspruch 1 in einer Lösung, die zwischen 0,5 % und 50 % PVP aufweist.

18. Formulierung nach Anspruch 17, bei der die Lösung etwa 5 % PVP enthält.

19. Nicht menschliches transgenes Lebewesen, das eine Vielzahl von Zellen aufweist, die den Vektor nach Anspruch 1 enthalten.

20. Transgenes Lebewesen nach Anspruch 19, bei dem die Zelle ein Bakterium oder eine somatische Zelle ist.

21. Zelle, die mit einem Vektor nach Anspruch 1 transformiert ist.

22. Transformierte Zelle nach Anspruch 21, wobei die Zelle myogen ist.

23. Verfahren zum Transfizieren einer Zelle ex vivo, mit dem Schritt, die Zelle über eine ausreichende Zeitspanne mit einem Vektor nach Anspruch 1 in Kontakt zu bringen, um die Zelle zu transfizieren.

24. Verfahren nach Anspruch 23, bei dem das Inkontaktbringen in Anwesenheit einer etwa 5 % PVP enthaltenden Lösung durchgeführt wird.

25. Verfahren nach Anspruch 23, wobei zu dem Verfahren ferner die Schritte gehören, den Vektor mit einem selektierbaren Marker zu co-transfizieren und die transformierten Zellen zu selektieren.

26. Verfahren zum Liefern und Exprimieren eines menschlichen IGF-I-Gens ex vivo in eine Vielzahl von Zellen, mit den Schritten:
(a) Transfizieren der Vielzahl von Zellen ex vivo mit einem Vektor nach Anspruch 1; und
(b) Inkubieren der Vielzahl von Zellen unter Bedingungen, die ein Exprimieren einer Nucleinsäuresequenz aus dem Vektor erlauben, wobei die Nucleinsäuresequenz für IGF-I kodiert.

27. Verfahren nach Anspruch 26, bei dem das IGF-I hIGF-I ist, und die Zellen menschliche Zellen sind.

28. Verfahren nach Anspruch 27, bei dem das Inkontaktringen in Anwesenheit einer etwa 5 % PVP enthaltenden Lösung durchgeführt wird.

29. Verwenden eines Vektors nach Anspruch 1, der geeignet ist, eine Zelle in Form eines Medikaments in situ zu transfizieren.

30. Verwenden eines Vektors nach Anspruch 1 bei der Herstellung eines Medikaments für die Behandlung einer Erkrankung oder eines Zustands, ausgewählt aus der Gruppe, zu der Muskelatrophie, Osteoporose, Diabetes, Neuropathie und Wachstumsstörungen gehören.

31. Verfahren nach Anspruch 30, bei dem die Erkrankung oder der Zustand eine Muskelatrophie ist, die in Folge einer Schädigung oder Inaktivität des unteren Motoneurons auftritt.

## Revendications

1. Vecteur destiné à l'expression d'une séquence d'acide nucléique dans une cellule, comprenant :
une cassette d'acide nucléique contenant une séquence nucléotidique codant pour l'IGF-I ;
une région adjacente en 5' incluant une ou plusieurs séquences nécessaires pour l'expression de ladite cassette d'acide nucléique, où lesdites séquences incluent un promoteur d'un gène de l'α-actine squelettique ;
une séquence de liaison reliant ladite région adjacente en 5' à un acide nucléique, ladite séquence de liaison possédant une position permettant l'insertion de ladite cassette d'acide nucléique, ladite séquence de liaison n'étant naturellement pas associée aux régions en 5' et en 3' ; et
une région adjacente en 3' incluant une région non traduite (UTR) en 3' et éventuellement une région non codante (NCR) en 3', ladite région adjacente en 3' étant en 3' de ladite position permettant l'insertion de ladite cassette d'acide nucléique, et ladite région non traduite (UTR) en 3' comprenant une séquence fonctionnelle d'une région non traduite (UTR) en 3' de l'hormone de croissance.

2. Vecteur selon la revendication 1 où ledit IGF-I est un IGF-I humain.

3. Vecteur selon la revendication 2 où ladite séquence nucléotidique codant pour l'IGF-I humain est une séquence synthétique.

4. Vecteur selon la revendication 3 où ladite séquence nucléotidique codant pour l'IGF-I humain possède la séquence de la SEQ ID N°4.

5. Vecteur selon la revendication 1 où ledit promoteur d'un gène de l'α-actine squelettique est originaire du poulet.

6. Vecteur selon la revendication 1 où ledit promoteur d'un gène de l'α-actine squelettique est d'origine humaine.

7. Vecteur selon la revendication 1 où ladite région non traduite (UTR) en 3' de l'hormone de croissance provient d'un gène de l'hormone de croissance humaine.

8. Vecteur selon la revendication 1 où la répétition ALU ou une séquence du type répétition ALU est supprimée de ladite région non traduite (UTR) en 3'.

9. Vecteur selon la revendication 1, où ledit IGF-I est un IGF-I humain, ledit promoteur d'un gène de l'α-actine squelettique est originaire du poulet et ladite région non traduite (UTR) en 3' de l'hormone de croissance provient d'un gène de l'hormone de croissance humaine.

10. Vecteur selon la revendication 1 où ladite région adjacente en 5' ou ladite région adjacente en 3', ou les deux, régulent l'expression de ladite cassette d'acide nucléique principalement dans un tissu spécifique.

11. Vecteur selon la revendication 2 où ledit tissu spécifique est myogénique.

12. Vecteur selon la revendication 1 où ladite région adjacente en 5' inclut un promoteur, une boîte TATA, un site Cap et un premier intron et une jonction dans une relation appropriée pour l'expression de ladite cassette d'acide nucléique.

13. Vecteur selon la revendication 12 où ladite région adjacente en 5' comprend en outre une séquence d'ARNm leader en 5' insérée entre ledit promoteur et ladite cassette d'acide nucléique.

14. Vecteur selon la revendication 1 où ledit vecteur comprend en outre un intron/région non traduite (UTR) en 5' du gène d'une α-actine squelettique du poulet.

15. Vecteur selon la revendication 1 où ledit vecteur comprend en outre un gène de résistance à un antibiotique.

16. Vecteur selon la revendication 1 où ledit vecteur comprend une séquence nucléotidique qui est la même que la séquence nucléotidique du plasmide pIG0552 telle que montrée dans le tableau 1.

17. Formulation destinée à la distribution et à l'expression d'un gène de l'IGF-I humain dans une cellule, ladite formulation comprenant
un vecteur selon la revendication 1 dans une solution contenant entre 0,5% et 50% de PVP.

18. Formulation selon la revendication 17, ladite solution contenant environ 5% de PVP.

19. Animal transgénique non humain possédant une pluralité de cellules contenant le vecteur de la revendication 1.

20. Animal transgénique selon la revendication 19 où ladite cellule est une cellule germinale ou somatique.

21. Cellule transformée avec un vecteur selon la revendication 1.

22. Cellule transformée selon la revendication 21, ladite cellule étant myogénique.

23. Procédé de transfection d'une cellule *ex vivo,* comprenant l'étape consistant à mettre en contact ladite cellule avec un vecteur selon la revendication 1 pendant une durée suffisante pour transfecter ladite cellule.

24. Procédé selon la revendication 23 où ladite mise en contact est effectuée en présence d'une solution de PVP à environ 5%.

25. Procédé selon la revendication 23, comprenant en outre les étapes de cotransfection dudit vecteur avec un marqueur sélectionnable et la sélection des cellules transformées.

26. Procédé de distribution et d'expression *ex vivo* d'un gène de l'IGF-I humain dans une pluralité de cellules, comprenant les étapes consistant à
(a) transfecter ladite pluralité de cellules ex vivo avec un vecteur selon la revendication 1 ; et
(b) incuber ladite pluralité de cellules dans des conditions permettant l'expression d'une séquence d'acide nucléique dans ledit vecteur, ladite séquence d'acide nucléique codant pour l'IGF-I.

27. Procédé selon la revendication 26, où ledit IGF-I est l'IGF-I-h et lesdites cellules sont des cellules humaines.

28. Procédé selon la revendication 27, où ladite mise en contact est effectuée en présence d'une solution de PVP à environ 5%.

29. Utilisation d'un vecteur selon la revendication 1 qui est approprié pour la transfection d'une cellule *in situ,* en tant que médicament.

30. Utilisation d'un vecteur selon la revendication 1 dans la fabrication d'un médicament destiné au traitement d'une maladie ou d'un état sélectionné au sein du groupe formé par l'atrophie musculaire, l'ostéoporose, le diabète, la neuropathie et les troubles de la croissance.

31. Utilisation selon la revendication 30, ladite maladie ou état étant une atrophie musculaire secondaire à une lésion ou une maladie des neurones moteurs inférieurs.
